# EUROPEAN PATENT APPLICATION

(11) **EP 0 739 984 A1**
(43) Date of publication of application: **30.10.1996**
(21) Application number: 95106275.1
(22) Date of filing: 26.04.1995
(51) Int. Cl.: C12N 15/62, C07K 19/00, C07K 14/46

(54) **Bivalent polypeptides containing at least two domains**

(71) Applicant: SAN TUMORFORSCHUNGS-GMBH, D-79106 Freiburg i. Br. (DE)
(72) Inventor: Wels, Winfried Dr., D-79312 Emmendingen (DE); Schmidt, Mathias, D-79115 Freiburg (DE); Groner, Bernd, Dr., CH-4059 Basel (CH)
(74) Representative: Weiss, Wolfgang, Dipl.-Chem. Dr.

(57) **Abstract**

The present invention is directed to polypeptides useful in inhibiting the growth of certain tumor cells, nucleic acids encoding said polypeptides and pharmaceutical compositions comprising said polypeptides as an active substance.

## Description

### Field of the Invention

The present invention is directed to polypeptides useful in inhibiting the growth of certain tumor cells, nucleic acids encoding said polypeptides and pharmaceutical compositions comprising said polypeptides as an active substance.

### Background of the Invention

Recent research has uncovered the important role played by the growth factor receptor tyrosine kinases in the etiology and development of human malignancies. These biological receptors are anchored by means of a transmembrane domain in the membranes of cells that express them. An extracellular domain binds to a growth factor. The binding of the growth factor to the extracellular domain results in a signal being transmited to the intracellular kinase domain. The transduction of this signal is responsible for the proliferation and differentiation of the cells.

Members of the epidermal growth factor (EGF) receptor family are important growth factor receptor tyrosine kinases. The first member of the EGF receptor family of growth factors receptor to be discovered was a glycoprotein having an apparent molecular weight of approximately 165 kD. This glycoprotein, which was described by Mendelsohn et al. in U.S. patent 4,943,533, is known as the EGF receptor. The binding of EGF or transforming growth factor alpha (TGF-alpha) to the EGF receptor leads to cell growth.

Another member of the EGF receptor family of growth factor receptors is called erbB-2, also known as HER2 or p185 (Coussens et al., Science 230 (1985), 1132-1139; Yamamoto et al., Nature 319 (1986), 230-234). erbB-2, which is expressed by the c-erbB-2 gene, is identical with the protein expressed by the human neu oncogene. The amino acid sequence of erbB-2 is different from, but highly homologous to, that of the EGF receptor. Other members of the EGF receptor family include erbB-3/HER-3 (Kraus et al., Proc.Natl.Acad.Sci. USA 86 (1989), 9193-9197; Plowman et al., Proc.Natl.Acad.Sci. USA 87 (1990), 4905-4909) and erbB-4/HER-4 (Plowman et al., Proc.Natl.Acad.Sci. USA 90 (1993), 1746-1750).

Many receptor tyrosine kinases are found in unusually high numbers on human tumors. For example, many tumors of epithelial origin express increased levels of EGF receptor on their cell membranes. Examples of tumors that express EGF receptors include glioblastomas as well as cancers of the lung, breast, head and neck, and bladder. The c-erbB-2/neu gene is amplified and/or overexpressed in a number of human adenocarcinomas arising at various sites, including breast, ovary, lung, stomach, and salivary glands. The amplification and/or overexpression of the EGF and erbB-2 receptors on the membranes of tumor cells is associated with a poor prognosis.

Antibodies, especially monoclonal antibodies, raised against tumor antigens have been investigated as potential anti-tumor agents. Such antibodies may inhibit the growth of tumors through a number of mechanisms. For example, antibodies may inhibit the growth of tumors immunologically through antibody-dependent cellular cytoxicity (ADCC) or complement-dependent cytoxicity (CDC).

Alternatively, antibodies may compete with the binding of a growth factor to its receptor, thereby inhibiting the growth of tumors that express the receptor. In another approach toxins are conjugated to antibodies raised against tumor antigens. The antibody portion directs the conjugate to the tumor, which is killed by the toxin portion.

For example, U S. patent 4,943,533 describes two murine monoclonal antibodies that bind to the EGF receptor. The antibodies are called 225 and 528. The patent is assigned to the University of California and licensed exclusively to ImClone Systems Incorporated. International patent application W089/06692 of Genentech discloses murine monoclonal antibodies specific for the c-erbB-2 gene product.

A disadvantage of murine monoclonal antibodies is the human anti-mouse antibody (HAMA)response due to the presence of mouse Ig sequences. This disadvantage can be minimized by replacing the entire constant region of a murine (or other non-human mammalian) antibody. Replacement of the constant regions of a murine antibody with human sequences is usually referred to as chimerization.

The chimerization process can be made even more effective by also replacing the variable regions other than the complementarity-determining regions (CDRs) of a murine antibody with the corresponding human sequences. The replacement of the constant regions and non-CDR regions with human sequences is usually referred to as humanization. The humanized antibody is less immunogenic the more murine sequences are replaced by human sequences. Unfortunately, both the cost and effort increase as more regions of a murine antibodies are replaced by human sequences.

Another approach to reducing the immunogenicity of antibodies is the use of antibody fragments. For example, an article by Aboud-Pirak et al., Journal of the National Cancer Institute 80, 1605-1611 (1988), compares the efficacy of an anti-EGF receptor antibody called 108.4 with fragments of the antibody as anti-tumor agents. The tumor model was based on KB cells as xenografts in nude mice. KB cells are derived from human oral epidermoid carcinomas, and express elevated levels of EGF receptors.

Aboud-Pirak et al. found that both the antibody and the bivalent F(ab)'₂ fragment retarded tumor growth in vivo, although the F(ab)'₂ fragment was less efficient. The monovalent Fab fragment of the antibody, whose ability to bind to the cell-associated receptor was conserved, did not, however, retard tumor growth at all.

A contrary conclusion was reached by Mueller et al. in Cancer Research 51, 2193-2198 (1991). These authors treated established subcutaneous M24met tumors in SCID mice with an anti-EGF antibody called 425.3. M24 met is a melanoma cell line that metastasizes spontaneously from subcutaneous tumors to multiple distant sites in SCID mice.

Mueller et al. reported that the antibodies specifically suppress spontaneous metastasis of these tumors. Interestingly, however, treatment of the SCID mice with the F(ab)'₂ fragment of the 425.3 antibody did not affect M24met metastasis.

Although the use of antibodies and antibody fragments as anti-tumor agents has shown promise, there are disadvantages that must be overcome. For example, monoclonal antibodies are produced in hybridomas. The use of hybridomas in commercial production is, however, less efficient than that of other expression systems such as bacteria. Moreover, the monoclonal antibodies are normally generated in animals other than humans. Such monoclonal antibodies are immunogenic in humans. This immunogenicity limits the effectiveness of the treatment of humans with such monoclonal antibodies.

The problem of immunogenicity can be overcome by means of chimerization and humanization techniques discussed above. While one can easily replace most amino acid sequences, especially constant region sequences, with equivalent human sequences, the presence of the remaining non-human sequences, including variable region sequences essential for binding, still causes immunogenicity. The more non-essential amino acids sequences one replaces with human sequences, the more difficult and expensive the procedure becomes, and the more unpredictable the results are.

The difficulties in replacing the non-human amino acid sequences of an antibody with human sequences can be avoided by producing antibody fragments, such as monovalent (one binding site) Fab fragments and bivalent (two binding sites) F(ab')₂ fragments. The production of such fragments, however, requires adding steps to the process for producing monoclonal antibodies. These steps include enzymatic digestion, fragment isolation and final purification.

The above problems relating to immunogenicity and production difficulty can be alleviated by producing recombinant DNA molecules encoding only the CDR regions of an antibody. Such molecules are sometimes called Fv molecules, and may be produced by expression of DNA encoding the hypervariable domains of the heavy and light chains of an antibody joined by a short peptide linker. See International Patent Application W089/09825 (CellTech). Such Fv molecules are easy to produce and have little immunogenicity. Moreover, their small size permits them to invade solid tumors more deeply.

A further advantage of Fv molecules is that the DNA encoding the Fv molecule may also encode an effector molecule, such as a toxin and/or a signal sequence directing the expressed polypeptide to a particular compartment of the cell or for facilitating purification.

For example, Wels et al., European patent application 502 812 (Ciba-Geigy) disclose single chain antibodies that bind specifically to the extracellular domain of growth factor receptor erbB-2 (gpl85). The single chain antibody contains the heavy chain variable domain and the light chain variable domain joined by means of a spacer peptide. The single chain antibody is linked to an effector molecule. Examples of effector molecules said to be those useful for diagnostic purposes include enzymes that cause a detectable reaction. Examples of effector molecules said to be useful for therapeutic purposes include toxins. Among the enzymes said to be useful for causing detectable reactions are alkaline phosphatase, horseradish peroxidase, beta-D-galactosidase, or a peptide having a particular binding property, for example, streptavidin. Toxins said to be useful therapeutically are said to include cytolysin or an exotoxin such as ricin A, diphtheria toxin, or Pseudomonas exotoxin. See page 3, line 21 et seq. of EP 502 812.

While single chain antibodies are able to inhibit the growth of tumor cells by means of some of the mechanisms attributed to monoclonal antibodies, they are unable to do so by means of all such mechanisms. For example, bivalent monoclonal antibodies, as well as growth factors, such as EGF and TGF-alpha, are able to induce dimerization or other aggregation of the receptors. Single chain antibodies, however, are monovalent, and are unable to do so. Suggestions have been made that the dimerization of growth factor receptors is responsible for receptor activation (i.e. phosphorylation), and that receptor activation is associated with internalization of the receptor. Such dimerization includes the formation of homodimers of EGF and erbB-2 receptors and heterodimers between the EGF and erbB-2 receptors. See Harwerth et al., Journal of Biological Chemistry 267, 15160-15167 (1992).

There is, therefore, a continuing need for improved anti-tumor agents that can be efficiently and inexpensively produced, have little or no immunogenicity in humans, and are capable of forming homodimers or heterodimers of receptors that are expressed in high numbers on tumor cells. An object of the present invention is the discovery of such new anti-tumor agents that combine the advantageous features of monoclonal antibodies, antibody fragments and single chain antibodies.

### Summary of the Invention

These and other objects as will be apparent from the foregoing have been achieved by producing new recombinant fusion polypeptides which have a high potential as anti-tumor agents. In a first aspect, the present invention refers to a recombinant fusion polypeptide comprising at least two independent cell surface binding domains, at least one of which binds to a member of the EGF receptor family of growth factor receptors. In a second aspect, the present invention refers to a recombinant fusion polypeptide comprising at least two independent cell surface binding domains and at least one effector region, preferably a cytotoxic domain.

The invention also includes DNA encoding such polypeptides, vectors containing such DNA, host cells capable of expressing such DNA, pharmaceutical compositions comprising such polypeptides as an active substance and methods for inhibiting the growth of tumors comprising treatment with such polypeptide.

### Detailed Description of the Invention

The polypeptide according to the present invention is a recombinant fusion polypeptide which is encoded by a single DNA sequence. This polypeptide comprises at least two independent cell surface binding domains which can be the same or different. The binding domains can bind to the same molecules on the cell surface or not. Preferably, the binding domains are different. More preferably, the binding domains recognize different molecules on the surface of a cell. Further it is preferred that each binding domain is able to bind to a cell surface even in the absence of the respective other binding domain.

The cell surface binding domains recognize a molecule on the surface of a cell, preferably a eukaryotic cell, more preferably, a vertebrate cell and, most preferably, a mammal cell, e.g. a human cell. The binding is specific for a defined structure on the cell surface. Binding also takes place with high affinity, preferably with a binding constant of at least 10⁻⁶ M.

The respective independent binding domains of the fusion polypeptide and other domains, if present, are preferably separated by a peptide spacer which comprises 2-50, more preferably 5-25 and, most preferably, about 15 amino acids.

In the first aspect of the present invention a polypeptide is provided comprising at least two, preferably two, three or four independent binding domains, at least one of which binds to a member the EGF receptor family of growth factor receptors, e.g. the EGF receptor, erbB-2, erbB-3 or erbB-4. Preferably, the polypeptide comprises at least two binding domains which are specific for members of the EGF receptor family. More preferably, these binding domains bind to different members of the EGF receptor family.

Optionally, the polypeptide comprises further binding domains. These further binding domains can also bind to members of the EGF receptor family or alternatively to different molecules on the cell surface, preferably, other cytokine receptors, preferably cytokine receptors which upon ligand binding are capable of being internalized into the cytoplasm. Examples of such cytokine receptors are cytokine receptor tyrosine kinases such as other growth factor receptor tyrosine kinases. Examples of receptor tyrosine kinases are the insulin receptor (Ganguli, S. et al., Curr.Top.Cell.Regulat. 27, 38 (1985)), the colony stimulating factor 1 (CSF-1) receptor (Ullrich, A. et al., Nature 309, 418 (1984)) or a member of the fibroblast growth factor (FGF) receptor family (Lee, P.L., Science 245, 57 (1989)).

Alternatively, these further binding domains can also bind to surface molecules which are not receptors for growth factors or cytokines but display selective and/or enhanced expression on tumor cells. Examples of such surface molecules are the CD24 molecule which is expressed on small cell lung carcinoma cells and which is recognized by the murine monoclonal antibody SWA11 (Jackson et al., Cancer Res.52, 5264 (1992)). Other examples are a new epitope on the neural cell adhesion molecule recognized by the murine monoclonal antibody SEN7 (Waibel et al., Cancer Res.53, 2840 (1993)), the 38 kDa pancarcinoma antigen recognized by the monoclonal antibody MOC-31 (de Jonge et al., European J.Cancer 29A, 1885 (1993)), and the cluster-5A small cell lung carcinoma antigen recognized by the murine monoclonal antibody SWA20 (Waibel et al., Br.J.Cancer 63, Suppl.XIV, 29 (1991)).

The second aspect of the present invention refers to a polypeptide comprising at least two independent cell surface binding domains and further at least one effector region, e.g. one effector region for each binding domain. Preferably, these binding domains can bind to members of the EGF receptor family or to other cytokine receptors as defined above. Examples of effector regions are detectable molecules for diagnostic purposes, such as enzymes or peptides having a particular binding property, e.g. streptavidin. Preferably, the effector region comprises a cytotoxin, i.e. a polypeptide which can inhibit cell growth or cause the destruction of a cell. The cytotoxin can be a bacterial cytotoxin, such as diphtheria toxin derived from Corynebacterium diphtheriae, exotoxin A derived from Pseudomonas aeruginosa, anthrax toxins derived from Bacillus anthracis, Shigatoxin derived from Shigella, Shiga-like toxins derived from Escherichia coli, or pertussis toxins derived from Bordatella pertussis or a toxic region thereof. Preferably, a bacterial cytotoxin is exotoxin A from Pseudomonas aeruginosa (ATCC 25313-25363) or a toxic region thereof, e.g. the domains II, Ib and/or III.

On the other hand, the cytotoxin can also be derived from plants. Examples of plant cytotoxins are ricin (Lamb et al., Eur.J.Biochem. 148, 265-270 (1985)), abrin (Wood et al., Eur.J.Biochem. 198, 723-732 (1991)), a ribozyme inactivating protein, such as saporin (Benatti et al., Eur.J.Biochem. 183, 465-470 (1989)), pokeweed antiviral protein (Kataoka et al., Plant Mol.Biol.20, 879-886 (1992)), gelonin (Nolan et al., Gene 134, 223-227 (1993)) or trichosanthin (Shaw et al., Gene 97, 267-272 (1991)).

In a further preferred embodiment the effector region comprises a ribonuclease, particularly a ribonuclease derived from a mammal, e.g. bovine pancreatic RNase A (Carsana et al., Nucleic Acids Res. 16, 5491-5502 (1988)), human angiogenin (Kurachi et al., Biochemistry 24, 5494-5499 (1985)) or human eosinophil-derived neurotoxin (Rosenberg et al., Proc.Natl.Acad.Sci. USA 86, 4460-4464 (1989)). Most preferably, the ribonuclease is derived from a human.

A binding domain of the polypeptide can comprise a single-chain binding region of an antibody, which is preferably the variable heavy-chain sequence and the variable light-chain sequence of the antibody connected by a peptide spacer which comprises 2-50, more particularly 5-25 and especially about 15 amino acids. Preferably, the antibody recognizes a member of the EGF family of growth factor receptors or another cytokine receptor, e.g. a receptor tyrosine kinase. Examples of antibodies having suitable binding regions are the antibody 225 (U.S.-Patent 4,943,533) which binds to the EGF receptor. Other examples are the binding regions of the monoclonal antibodies FRP5, FSP16, FSP77 or FWP51 disclosed in WO 89/06692, which are specific for the erbB-2 receptor. Hybridoma cell lines producing the antibodies FRP5, FSP16, FSP77 and FwP51 have been deposited at the European Collection of Animal Cell Cultures (ECACC) under the accession numbers 90112115, 90112116, 90112117 and 90112118, respectively.

In another embodiment of the present invention at least one binding domain comprises the binding region of a cytokine, e.g. a growth factor, more preferably a cytokine which binds to a receptor tyrosine kinase. Examples of suitable growth factors are growth factors of the EGF family, such as EGF (Xu et al., Nature 309, 806-810 (1984); Bell et al., Nucleic Acids Res.14, 8427-8446 (1986)), TGFα (Qian et al., Gene 132, 291-296 (1993)), neu differentiation factor or variants thereof (Wen et al., Cell 69, 559-572 (1992) and Mol.Cell.Biol.14, 1909-1919 (1994)), amphiregulin (Plowman et al., Mol.Cell.Biol.10, 1969-1981 (1990)), heparin-binding EGF (Higashijama et al., Science 251, 936-939 (1991)), heregulin and isoforms thereof (Holmes et al., Science 256, 1205-1210 (1992)) or cripto-1 (Ciccodicola et al., EMBO J.8, 1987-1991 (1989)). Especially preferred are the binding regions of EGF or TGFα.

The polypeptide according to the present invention can comprise at least two antibody binding regions which can be derived from antibodies which are directed to the same molecule or different molecules. On the other hand, the polypeptide according to the present invention can comprise at least two binding regions of cytokines, e.g. growth factors.

In an especially preferred embodiment at least one binding domain comprises the binding region of an antibody and at least one binding domain comprises the binding region of a cytokine, e.g. a growth factor. According to the present invention several recombinant polypeptides having at least two independent binding domains have been produced. Specific examples are polypeptides comprising two single-chain antibody binding regions from the antibodies FRP5 and 225, respectively, fused to fragments of exotoxin A of P.aeruginosa. Another specific example is a polypeptide comprising a single-chain binding region of the antibody FRP5 and the receptor binding region of TGFα fused to fragments of exotoxin A. Still a further specific example is a recombinant polypeptide comprising two single-chain binding regions of the monoclonal antibody FRP5 fused to fragments of exotoxin A. Binding of these bivalent, bispecific polypeptides to target cells can result in a much higher cell-killing activity than the corresponding monospecific polypeptides (comprising only one binding domain). Surprisingly, the bivalent, bispecific antibody-toxins can bind simultaneously to different growth-factor-receptors on the same tumor cell. Further they can induce the formation of receptor heterodimers and cause receptor activation. This in turn leads to receptor internalization, toxin uptake and enhanced cell killing. Therefore, the bispecific polypeptides according to the present invention have a high potential as reagents for the treatment of cancer.

Another subject matter of the present invention is a DNA molecule encoding a fusion polypeptide as defined above and a vector comprising at least one copy of the DNA.

The vector can be capable of replication in eukaryotes, prokaryotes or in eukaryotes and prokaryotes. It can be a vector capable of integration into the genome of the host cell (e.g. bacteriophage lambda) or a vector which is present extrachromosomally (e.g. a plasmid). Preferably, the vector is a plasmid.

Further, the vector can be an expression vector, i.e. a vector in which the DNA sequence is under control of a suitable regulatory sequence which allows expression, i.e. transcription and translation of the inserted DNA sequence. Suitable expression vectors for prokaryotic host cells are described in Sambrook et al., Molecular Cloning. A Laboratory Manual, 2nd Edition (1989), Cold Spring Harbor Laboratory Press, especially in Chapters 1-4 and 17. Suitable vectors for expression of cloned genes in mammalian cells are described in Sambrook et al., Chapter 16.

A further subject matter of the present invention is a host cell capable of expressing the DNA molecule as defined above, particularly a host cell which is stably transformed with an expression vector containing said DNA molecule. Preferably, the host cell is a bacterial cell, more preferably an E.coli cell.

Specific examples of expression vectors of the present invention are the E.coli vectors pMS238-5-TGF, pMS238-5-225 , pMS238-225-5, pMS240-5-225, pMS242-5-5 KDEL, pMS238-5-5 and pMS246-5-5 KDEL. The nucleotide sequences of plasmid fragments encoding polypeptides of the present invention and the corresponding amino acid sequences are shown in SEQ ID Nos.11-24 of the enclosed sequence listing.

A further subject matter of the present invention is a pharmaceutical composition comprising as an active substance a pharmaceutically effective amount of a polypeptide as defined above, optionally in combination with pharmaceutically acceptable carriers, diluents and adjuvants. This pharmaceutical composition is preferably used as an agent for the inhibition of cell proliferation, more preferably as an anti-tumor agent, i.e. an agent for inhibiting the growth of a tumor.

The pharmaceutical composition can be in any suitable form, e.g. in the form of a solution, suspension, powder, lyophilisate, ointment or tincture. The composition can be administered by any suitable method, e.g. per injection (systemically or locally) or topically.

The respective pharmaceutically effect amount can depend on the specific patient to be treated, on the disease to be treated and on the method of administration. Further, the pharmaceutically effective amount depends on the specific polypeptide used, especially if the polypeptide additionally contains a cytotoxic component or not. The treatment usually comprises a multiple administration of the pharmaceutical composition, usually in intervals of several hours, days or weeks. The pharmaceutically effective amount of a dosage unit of the polypeptide usually is in the range of 0.01 ng to 100 µg per kg of body weight of the patient to be treated.

The invention is further illustrated by the following sequence listings 1-24 and Examples.
- SEQ ID NO.1 and 2: show the nucleotide sequence of a fragment of the plasmid pWW152-225 encoding the single-chain binding region of the antibody 225 and the corresponding amino acid sequence.
- SEQ ID NO.3 and 4: show the nucleotide sequence of a fragment of the plasmid pWW152-5 encoding the single-chain binding region of the antibody FRP5 and the corresponding amino acid sequence.
- SEQ ID NO.5 and 6: show the nucleotide sequence of a fragment of the bacterial expression plasmid pSW202-225 encoding a fusion polypeptide of the single-chain binding region of the antibody 225 and a region of exotoxin A and the corresponding amino acid sequence.
- SEQ ID NO.7 and 8: show a nucleotide sequence of a fragment of the bacterial expression plasmid pSW202-5 encoding a fusion polypeptide of the single-chain binding region of the antibody FRP5 and a region of exotoxin A and the corresponding amino acid sequence.
- SEQ ID NO.9 and 10: show the nucleotide sequence of a fragment of the bacterial expression plasmid pSW202-TGF encoding a fusion polypeptide of TGFα and a region of exotoxin A and the corresponding amino acid sequence.
- SEQ ID NO.11 and 12: show the nucleotide sequence of a fragment of the bacterial expression plasmid pMS238-5-TGF encoding a fusion polypeptide of the single-chain binding region of the antibody FRP5, TGFα and regions of exotoxin A and the corresponding amino acid sequence.
- SEQ ID NO.13-18: show the nucleotide sequences of fragments of the bacterial expression plasmids pMS238-5-225,pMS238-225-5 and pMS240-5-225 encoding fusion polypeptides of the single-chain binding regions of the antibodies FRP5 and 225, respectively, and regions of exotoxin A and the corresponding amino acid sequences.
- SEQ ID NO.19-24: show the nucleotide sequences of fragments of the bacterial expression plasmids pMS242-5-5 KDEL, pMS238-5-5 and pMS246-5-5 KDEL, respectively, encoding fusion polypeptides of two single-chain binding regions of the antibody FRP5 and regions of exotoxin A and the corresponding amino acid sequences.

### Example 1. Construction of plasmid pWW152:

A pBluescript KS+ (Stratagene) derived plasmid is constructed to be used for the cloning of immunoglobulin heavy (VH) and light chain variable domain (VL) cDNAs and the assembly of VH and VL into a single-chain Fv fragment (scFv) encoding gene.
1.1 Oligonucleotides: A double stranded DNA adaptor with HindIII and PstI compatible ends is constructed by annealing 0.5 nmol of the oligonucleotide 5'-AGCTTCAGGTACAACTGCA-3' with 0.5 nmol of the oligonucleotide 5'-GTTGTACCTGA-3' by incubation at 65°C for 3 minutes and cooling to room temperature.
1.2 Derivation of pWW15 vector fragment and purification:
   pWW15 (1 µg) which is a plasmid containing a multiple cloning site and a synthetic DNA insert encoding the 15 amino acids sequence (GlyGlyGlyGlySer)₃ for the connection of VH and VL sequences (Wels et al., Biotechnology 10: 1128, 1992) is digested with HindIII and PstI. DNA fragments are separated on a 1.0% (w/v) agarose gel (ultra pure agarose, BRL) and the expected 3.1 kb HindIII/PstI vector fragment is eluted using the QIAquick gel extraction kit (QIAGEN) according to procedures provided by the manufacturer.
1.3 Ligation of pWW15 HindIII/PstI fragment and oligonucleotide adaptor: pWW15 (50 ng) HindIII/PstI fragment and 50 pmol oligonucleotide adaptor are ligated using 0.5 U T4 DNA ligase (New England Biolabs) in 50 mM Tris-HCl, pH 7.8, 10 mM magnesium chloride, 10 mM DTT, and 0.8 mM ATP overnight at 16°C. One half of ligation mixture is used to transform E.coli XL1 Blue (Stratagene) to obtain ampicillin resistant colonies. These are screened for the desired ligation product using a NaOH based plasmid "miniprep" method (Maniatis et al., Molecular Cloning: A Laboratory Manual / Second Edition, Cold Spring Harbor Laboratory, 1989). The following plasmid is obtained: pWW152.

### Example 2. Cloning of monoclonal antibody 225 heavy and light chain variable domain cDNAs

Total cellular RNA isolated from the hybridoma cell line producing the murine monoclonal antibody 225 specific for the human epidermal growth factor (EGF) receptor (Kawamoto et al., Proc. Natl. Acad. Sci. USA 80: 1337, 1983) provides the source for cDNA synthesis and subsequent amplification of monoclonal antibody 225 heavy and light chain variable domain encoding cDNA fragments. Amplification products of the expected size are purified from agarose gels and cloned into appropriate vectors. Intact cDNA clones are identified by sequencing. VH and VL cDNA domains are connected by gene fusion to obtain a scFv(225) encoding gene.
2.1 Isolation of total cellular RNA from 225 hybridoma cells:
   225 hybridoma cells (1 x 10⁸) are grown in suspension culture in RPMI 1640 (Seromed) further containing 20% FCS (fetal calf serum; Amined), 4 mM glutamine, 1 mM sodium pyruvate and 14.2 mM 2-mercaptoethanol in a humidified atmosphere of air and 7.5% CO₂ at 37°C. The cells are washed twice with PBS (phosphate buffered saline) on ice, PBS is removed and the cell pellet is kept on ice. Total RNA is extracted using the acid guanidinium thiocyanate-phenol-chloroform method described by Chomczynski & Sacchi (Anal. Biochem. 162: 156, 1987). The cells are lysed on ice in the presence of 10 ml denaturing solution (4 M guanidinium thiocyanate (Fluka), 25 mM sodium citrate, pH 7.0, 0.5% N-lauroylsarcosine (Sigma), 0.1 M 2- mercaptoethanol). The lysate is homogenized at room temperature. Sequentially, 1 ml of 2 M sodium acetate, pH 4.0, 10 ml of phenol (water saturated) and 2 ml of chloroform-isoamyl alcohol mixture (49:1) are added to the lysate. The final suspension is shaken vigorously for 10 sec and cooled on ice for 15 min. The samples are centrifuged at 10,000 x g for 20 min at 4°C. After centrifugation, RNA which is present in the aqueous phase is mixed with 10 ml of isopropanol and placed at -20°C for 1 h. The RNA precipitate is collected by centrifugation at 12,000 x g for 10 min at 4°C, the pellet dissolved in 3 ml water and the RNA precipitated by addition of 1 volume of isopropanol at -20°C. After centrifugation at 12,000 x g for 10 min at 4°C and washing the pellet in 70% ethanol, the final pellet of RNA is dissolved in water. The method yields approximately 300 µg of total cellular RNA. The final purified material is stored frozen at -20°C.
2.2 cDNA synthesis: 5 µg of total RNA isolated from 225 hybridoma cells is used in a 33 µl first strand cDNA synthesis reaction with 11 µl Bulk First-Strand Reaction Mix (Pharmacia), 200 ng NotI-d(T)₁₈ primer (Pharmacia), and 1 µl 200 mM DTT solution according to procedures provided by the manufacturer.
2.3 Polymerase chain reaction: 5 µl each of the cDNA reaction is used for DNA amplification in 2 independent 50 µl reactions containing either 25 pmol each of the two oligonucleotides complementary to regions in the mouse kappa light chain gene VKWlFOR 5'-GTTAGATCTCCARYTTKGTSCS-3' and VKlBACK (Orlandi et al., Proc. Natl. Acad. Sci. USA 86: 3833) 5'-GACATTCAGCTGACCCAGTCTCCA-3' (M=A+C, R=A+G, S=C+G, W=A+T, Y=C+T, K=G+T) or 25 pmol each of the two oligonucleotides complementary to regions in the mouse heavy chain gene VHlFOR (Orlandi et al., Proc. Natl. Acad. Sci. USA 86: 3833) 5'-TGAGGAGACGGTGACCGTGGTCCCTTGGCCCCAG-3' and VHlBACK (Orlandi et al., Proc. Natl. Acad. Sci. USA 86: 3833) 5'-AGGTSMARCTGCAGSAGTCWGG-3', 4 µl 2.5 mM dNTP (N= G, A, T, C) mixture, 5 µl 10x Taq DNA polymerase buffer (Boehringer Mannheim) and 2.5 U of Taq DNA polymerase (Boehringer Mannheim). Taq DNA polymerase is added after initial denaturation at 94°C for 2 min. For the first 5 cycles annealing is performed for 1 min at 37°C, for the following 25 cycles annealing is performed for 1 min at 62°C, primer extension for 1 min at 72°C, denaturation for 1 min at 94°C. Finally, amplification is completed by a 3 min primer extension step at 72°C.
2.4 Derivation of VH and VL cDNA fragments and purification:
   Amplification products are separated on a 1.5% (w/v) agarose gel (ultra pure agarose, BRL) and DNA fragments of the expected size are eluted. Subsequently the cDNA fragments are digested with PstI and BstEII (VH) or PvuII and BglII (VL). The expected PstI/BstEII (VH) and PvuII/BglII (VL) cDNA fragments are separated on a 1.5% agarose gel and purified by elution from the gel.
2.5 Ligation and construction of pWW152-225: pWW152 (50 ng) digested with PstI and BstEII and 30 ng of purified PstI/BstEII digested VH amplification product are ligated. The resulting plasmid is digested with PvuII and BclI and purified by agarose gel electrophoresis and elution from the gel. 50 ng of the PvuII/BclI digested vector fragment and 30 ng of purified PvuII/BglII digested VL amplification product are ligated and used to transform E.coli XL1 Blue (Stratagene). The following plasmid containing the scFv(225) encoding gene is obtained: pWW152-225. The nucleotide sequence encoding a scFv (225) polypeptide and the corresponding amino acid sequence are shown in SEQ ID NO.1 and 2.
Example 3. Construction of plasmid pWW152-5
   A scFv(FRP5) gene encoding a scFv molecule derived from the murine monoclonal antibody FRP5 specific for the human erbB-2 (Neu/HER-2) growth factor receptor (Harwerth et al., J. Biol. Chem. 267: 15160, 1992) is modified to obtain a gene fragment that can be used for the construction of scFv-effector fusion genes.
3.1 Derivation of the scFv(FRP5) encoding DNA fragment and purification: pWW15-5 (1 µg) (Wels et al., Biotechnology 10: 1128, 1992) is digested with PstI and XbaI. DNA fragments are separated on a 1.0% (w/v) agarose gel (ultra pure agarose, BRL) and the expected scFv(FRP5) encoding PstI/XbaI DNA fragment is eluted.
3.2 Ligation: pWW152 (50 ng) digested with PstI and XbaI and 30 ng of purified PstI/XbaI digested scFv(FRP5) encoding DNA fragnent are ligated and used to transform E.coli XL1 Blue (Stratagene). The following plasmid is obtained: pWW152-5. The nucleotide sequence encoding a scFv (FRP5) polypeptide and the corresponding amino acid sequence are shown in SEQ ID NO.3 and 4.

### Example 4. Construction of plasmid pSW50:

A bacterial expression vector based on plasmid pFLAG-l (IBI Biochemicals) is constructed which allows the insertion of scFv genes and the inducible bacterial expression of scFv proteins fused to the C-terminus of a stretch of six histidine residues (His)₆. This His sequence is used for the purification of recombinant protein via affinity chromatography.
4.1 Oligonucleotides: A double stranded DNA adaptor encoding a cluster of 6 His residues with HindIII and XbaI compatible ends is constructed by annealing 0.5 nmol of the oligonucleotide 5'- AGCTGCACCATCATCACCATCACAAGCTTGAATTCT-3' with 0 5 nmol of the oligonucleotide
   5'-CTAGAGAATTCAAGCTTGTGATGGTGATGATGGTGC-3' by incubation at 65°C for 3 minutes and cooling to room temperature.
   Sequence of partially double stranded HindIII/XbaI adaptor: bp 1 to 4 HindIII compatible overhanging end; bp 24 to 29 HindIII restriction site; bp 30 to 35 EcoRI restriction site; bp 37 to 40 XbaI compatible overhanging end.
4.2 Ligation: pFLAG-l (IBI Biochemicals) (50 ng) digested with HindIII and XbaI and 20 pmol HindIII/XbaI oligonucleotide adaptor are ligated and used to transform E.coli XL1 Blue (Stratagene). The following plasmid is obtained: pSW50.

### Example 5. Construction of plasmids pSW50-5 and pSW50-225

Plasmids are constructed which facilitate the bacterial expression of scFv(225) and scFv(FRP5)
5.1 Derivation of the scFv(FRP5) and scFv(225) encoding DNA fragments: 1 µg of each of the 2 plasmids pWW152-5 and pWW152-225 is digested with HindIII and XbaI. DNA fragments are separated on a 1.0% (w/v) agarose gel (ultra pure agarose, BRL) and the expected scFv(FRP5) and scFv(225) encoding HindIII/XbaI DNA fragments are eluted.
5.2 Ligation: pSW50 (50 ng) digested with HindIII and XbaI and 30 ng of either purified HindIII/XbaI digested scFv(FRP5) encoding DNA fragment or purified HindIII/XbaI digested scFv(225) encoding DNA fragment are ligated in 2 independent reactions and used to transform E.coli XL1 Blue (Stratagene). The following plasmids are obtained: pSW50-5 and pSW50-225.

### Example 6. Construction of plasmid pSW200

A plasmid is constructed which contains the Pseudomonas aeruginosa PAK exotoxin A (ETA) gene (Gray et al., Proc. Natl. Acad. Sci. USA 81: 2645, 1984; Lory et al., J. Bacteriol. 170: 714, 1988) lacking the region encoding the original target cell binding domain of the toxin.
6.1 Oligonucleotides: A double stranded DNA adaptor encoding a cluster of 6 His residues with HindIII and XbaI compatible ends is constructed by annealing 0.5 nmol of the oligonucleotide 5'-AGCTTCTCGAGCAGATCTCTCTAGAGCACCATCATCACCATCAC -3' with 0.5 nmol of the oligonucleotide 5'-CTAGGTGATGGTGATGATGGTGCTCTAGAGAGATCTGCTCGAGA -3' by incubation at 65°C for 3 min and cooling to room temperature.
   Sequence of partially double stranded HindIII/XbaI adaptor: bp 1 to 4 HindIII compatible overhanging end; bp 45 to 48 XbaI compatible overhanging end.
6.2 Ligation: pFLAG-l (IBI Biochemicals) is digested with SalI and treated with the Klenow enzyme to create blunt ends; subsequently the linearized fragment is digested with XbaI. pWW20 (Wels et al., Cancer Res. 52: 6310, 1992) is digested with EcoRI and treated with the Klenow enzyme to create blunt ends; subsequently the linearized fragment is digested with XbaI. The blunt-ended XbaI Pseudomonas aeruginosa exotoxin A (ETA) gene fragment is purified by agarose gel electrophoresis and elution and inserted into the blunt-ended XbaI pFLAG-l vector. The resulting plasmid, pSG100, is digested with HindIII and XbaI. 50 ng of digested pSG100 and 20 pmol HindIII/XbaI oligonucleotide adaptor are ligated and used to transform E.coli XL1 Blue (Stratagene). The following plasmid is obtained: pSW200.

### Example 7. Construction of scFv(FRP5)-ETA and scfv(225)-ETA expression plasmids

Bacterial expression plasmids are constructed which are used for the inducible bacterial expression of scFv-ETA fusion proteins.
7.1 Derivation of the scFv(FRP5) and scFv(225) encoding DNA fragments: 1 µg of each of the 2 plasmids pSW50-5 and pSW50-225 is digested with NdeI and XbaI. DNA fragments are separated on a 1.0% (w/v) agarose gel (ultra pure agarose, BRL) and the expected scFv(FRP5) and scFv(225) encoding NdeI/XbaI DNA fragments containing also the ompA signal peptide, the FLAG epitope and the N-terminal 6 His encoding sequences are eluted.
7.2 Ligation: pSW200 (50 ng) digested with NdeI and XbaI and 30 ng of either purified NdeI/XbaI digested scFv(FRP5) encoding DNA fragment or purified NdeI/XbaI digested scFv(225) encoding DNA fragment are ligated in 2 independent reactions and used to transform E.coli XL1 Blue (Stratagene). The following plasmids are obtained: pSW202-225 and pSW202-5. The nucleotide sequences encoding scFv (225)-ETA and scFv (FRP5)-ETA fusion polypeptides, respectively, and the corresponding amino acid sequences are shown in SEQ ID NO.5-8.

### Example 8. Construction of a human transforming growth factor (TGF)-α-ETA expression plasmid (pSW202-TGF)

Total cellular RNA isolated from MDA-MB-468 human breast carcinoma cells (ATCC HTB 132) provides the source for cDNA synthesis and subsequent amplification of a human transforming growth factor (TGF)-α encoding cDNA fragment. Amplification products of the expected size are purified from agarose gels and cloned into appropriate vectors. Intact cDNA clones are identified by sequencing. The TGF-α encoding cDNA fragment is used for the construction of a TGFα-ETA fusion gene.
8.1 Isolation of total cellular RNA from MDA-MB-468 cells:
   MDA-MB-468 cells (ATCC HTB 132) are grown as monolyers on 10 cm tissue culture plates (Falcon) at 37°C in DMEM (Seromed) further containing 8% FCS (Amined) and 100 µg/ml of gentamycin (Seromed) in a humidified atmosphere of air and 7.5% CO₂. The cells are washed twice with PBS on ice, PBS is removed and the plates are kept on ice. Total RNA is extracted using the acid guanidinium thiocyanate-phenol-chloroform method described by Chomczynski & Sacchi (Anal. Biochem. 162: 156, 1987). The cells from 2 semi-confluent 10 cm tissue culture plates are lysed on ice in the presence of 2 ml denaturing solution (4 M guanidinium thiocyanate (Fluka), 25 mM sodium citrate, pH 7.0, 0.5% N-lauroylsarcosine (Sigma), 0.1 M 2-mercaptoethanol). The lysate is homogenized at room temperature. Sequentially, 0.2 ml of 2 M sodium acetate, pH 4.0, 2 ml of phenol (water saturated) and 0.4 ml of chloroform-isoamyl alcohol mixture (49:1) are added to the lysate. The final suspension is shaken vigorously for 10 sec and cooled on ice for 15 min. The samples are centrifuged at 10,000 x g for 20 min at 4°C. After centrifugation, RNA which is present in the aqueous phase is mixed with 2 ml of isopropanol and placed at -20°C for 1 h. The RNA precipitate is collected by centrifugation at 12,000 x g for 10 min at 4°C, the pellet dissolved in 0.5 ml water and the RNA precipitated by addition of 1 volume of isopropanol at -20°C. After centrifugation at 12,000 x g for 10 min at 4°C and washing the pellet in 70% ethanol, the final pellet of RNA is dissolved in water. The method yields approximately 100 µg of total cellular RNA. The final purified material is stored frozen at -20°C.
8.2 cDNA synthesis: 5 µg of total RNA isolated from MDA-MB-468 cells is used in a 33 µl first strand cDNA synthesis reaction with 11 µl Bulk First-Strand Reaction Mix (Pharmacia), 200 ng NotI-d(T)₁₈ primer (Pharmacia), and 1 µl 200 mM DTT solution according to procedures provided by the manufacturer.
8.3 Polymerase chain reaction: 2 µl of the cDNA reaction is used for DNA amplification in a 50 µl reaction containing 25 pmol each of the two oligonucleotides complementary to regions in the human TGF-α gene 5'-GACCCGAAGCTTGGTACCGGTGTGGTGTCCCATTTTAATG-3' and 5'-TTCTGGGAGCTCTCTAGAGAGGCCAGGAGGTCCGC -3', 4 µl 2.5 mM dNTP (N= G, A, T, C) mixture, and 5 µl 10x Vent DNA polymerase buffer (New England Biolabs) and 2.5 U of Vent DNA polymerase (New England Biolabs). Vent DNA polymerase is added after initial denaturation at 94°C for 4 min. For 30 cycles annealing is performed for 1 min at 52°C, primer extension for 45 sec at 72°C, denaturation for 1 min at 94°C. Finally, amplification is completed by a 2 min primer extension step at 72°C.
8.4 Modification and purification: Amplification products are separated on a 1.5% (w/v) agarose gel (ultra pure agarose, BRL), DNA of the expected size is eluted and subsequently digested with HindIII and XbaI. The expected 171 bp DNA fragment encoding amino acids 1 to 50 of human TGF-α is separated on a 1.5% agarose gel and purified by elution.
8.5 Ligation: pSW202-5 vector fragment (50 ng) digested with HindIII and XbaI, and 30 ng of purified amplification product are ligated and used to transform E.coli XL1 Blue (Stratagene). The following plasmid is obtained: pSW202-TGF. The nucleotide sequence encoding a TGFα-ETA fusion polypeptide and the corresponding amino acid sequence are shown in SEQ ID NO.9 and 10.

### Example 9. Modification of the ETA gene fragment

A modified version of the ETA gene lacking the region encoding the original cell binding domain of the toxin is constructed which allows the insertion of foreign DNA sequences at different positions within the ETA sequence.
9.1 Derivation of DNA fragments and purification: pWW20 (1 µg) carrying a truncated ETA gene encoding amino acids 252 to 613 of exotoxin A from Pseudomonas aeruginosa PAK described by Wels et al. (Cancer Res. 52: 6310, 1992) is digested with XbaI and XhoI. DNA fragments are separated on a 1.0% (w/v) agarose gel (ultra pure agarose, BRL) and the expected 769 bp XbaI/XhoI DNA fragment encoding ETA amino acids 252 to 506 is eluted. The eluted fragment is subsequently digested with MaeII, DNA fragments are separated on a 1.5% (w/v) agarose gel and the expected 349 bp XbaI/MaeII DNA fragment encoding ETA amino acids 252 to 366 (designated ETA₂₅₂₋₃₆₆) is eluted.
9.2 Oligonucleotides: A double stranded DNA adaptor with MaeII and EcoRI compatible ends is constructed by annealing 0.5 nmol of the oligonucleotide 5'-CGAGAAGCTTGAGAGCTCTGACTACAAAGACGAACTTTAAG -3' with 0.5 nmol of the oligonucleotide 5'-AATTCTTAAAGTTCGTCTTTGTAGTCAGAGCTCTCAAGCTTCT -3' by incubation at 65°C for 3 min and cooling to room temperature.
   Sequence of partially double stranded MaeII/EcoRI adaptor: bp 1 to 2 MaeII compatible overhanging end; bp 5 to 10 HindIII restriction site; bp 13 to 18 SacI restriction site; bp 42 to 45 EcoRI compatible overhanging end.
9.3 Ligation: pWWl91 (50 ng), a pUCl9 derived plasmid where the original HindIII restriction site of the multiple cloning site of pUCl9 is destroyed and converted into a XbaI restriction site, digested with XbaI and EcoRI. and 30 ng of purified XbaI/MaeII ETA fragment, and 20 pmol MaeII/EcoRI oligonucleotide adaptor are ligated and used to transform E.coli XL1 Blue (Stratagene). The following plasmid containing an ETA gene fragment encoding ETA amino acids 252 to 366 is obtained: pWW25.
9.4 Polymerase chain reaction: 25 ng of pWW20 (Wels et al., Cancer Res.52:6310, 1992) is used for DNA amplification in a 50 µl reaction containing 50 pmol each of the two oligonucleotides complementary to regions in the ETA gene 5'-CTCGGGAGCTCTGGCGACGTCAG -3' and 5'- GCGGGGATCCGGCCTGCGTCGGG -3', 4 µl 2.5 mM dNTP (N= G, A, T, C) mixture, 5 µl 10x Taq DNA polymerase buffer (Boehringer Mannheim) and 2.5 U of Taq DNA polymerase (Boehringer Mannheim). Taq DNA polymerase is added after initial denaturation at 94°C for 2 min. For the first 5 cycles annealing is performed for 1 min at 42°C, for the following 25 cycles annealing is performed for 1 min at 55°C, primer extension for 1 min at 72°C, denaturation for 1 min at 94°C. Finally, amplification is completed by a 3 min primer extension step at 72°C.
9.5 Derivation of the ETA DNA fragments and purification:
   Amplification products are separated on a 1.2% (w/v) agarose gel (ultra pure agarose, BRL), DNA of the expected size is eluted and subsequently digested with SacI and BamHI. The expected SacI/BamHI ETA DNA fragment is separated on a 1.2% agarose gel and purified by elution. Plasmid pWW20 is digested with BamHI and EcoRI, DNA fragments are separated on a 1.2% (w/v) agarose gel (ultra pure agarose, BRL) and the expected BamHI/EcoRI ETA DNA fragment is eluted.
9.6 Ligation: pWW25 (50 ng) digested with SacI and EcoRI, 30 ng of purified BamHI/EcoRI ETA DNA fragment and 30 ng of purified SacI/BamHI digested ETA amplification product are ligated and used to transform E.coli XL1 Blue (Stratagene). The following plasmid containing an ETA gene fragment encoding ETA amino acids 252 to 366, 405 to 489, and 491 to 613 is obtained: pWW23.
9.7 Polymerase chain reaction: 25 ng of pWW20 (Wels et al., Cancer Res.52:6310, 1992) is used for DNA amplification in a 50 µl reaction containing 50 pmol each of the two oligonucleotides complementary to regions in the ETA gene 5'-GCCGGGAGCTCTGCGGGCCCGGCGG -3' and 5'-TTGCAGATCTTGCCAGATGCGTCGGGTTCCTG -3', 4 µl 2.5 mM dNTP (N= G, A, T, C) mixture, 5 µl 10x Taq DNA polymerase buffer (Boehringer Mannheim) and 2.5 U of Taq DNA polymerase (Boehringer Mannheim). Taq DNA polymerase is added after initial denaturation at 94°C for 2 min. For the first five cycles annealing is performed for 1 min at 42°C, for the following 25 cycles annealing is performed for 1 min at 55°C, primer extension for 1 min at 72°C, denaturation for 1 min at 94°C. Finally, amplification is completed by a 3 min primer extension step at 72°C.
9.8 Derivation of the ETA DNA fragments and purification:
   Amplification products are separated on a 1 2% (w/v) agarose gel (ultra pure agarose, BRL), DNA of the expected size is eluted and subsequently digested with SacI and BglII. The expected SacI/BglII ETA DNA fragment is separated on a 1.2% agarose gel and purified by elution.
9.9 Ligation: pWW23 (50 ng) digested with SacI and BamHI and 30 ng of purified SacI/BglII digested ETA amplification product are ligated and used to transform E.coli XL1 Blue (Stratagene). The following plasmid containing an ETA gene fragment encoding ETA amino acids 252 to 366 and 380 to 613 is obtained: pWW38.

### Example 10. Construction of a plasmid for the inducible bacterial expression of the bispecific scFv(FRP5)/TGFα-ETA (pMS238-5-TGF)

10.1 Derivation of the TGFα cDNA fragment: The amplification products from Example 8.3 are separated on a 1.2% (w/v) agarose gel (ultra pure agarose, BRL), DNA of the expected size is eluted and subsequently digested with HindIII and SacI. The expected HindIII/SacI TGFα cDNA fragment is separated on a 1.5% agarose gel and purified by elution from the gel.
10.2 Construction of pMS238-5-TGF: pWW38 (50 ng) digested with HindIII and SacI and 30 ng of purified HindIII/SacI digested TGFα amplification product are ligated. The resulting plasmid is digested with SalI and AatII. A 438 bp SalI/AatII fragment is purified by agarose gel electrophoresis and elution from the gel. pSW202-5 (50 ng) digested with SalI and AatII and 30 ng of purified 438 bp SalI/AatII fragment are ligated and used to transform E.coli XL1 Blue (Stratagene). The following plasmid is obtained: pMS238-5-TGF. The nucleotide sequence encoding a scFv (FRP5)-TGFα-ETA fusion polypeptide and the corresponding amino acid sequence are shown in SEQ ID NO.11 and 12.

### Example 11. Construction of a plasmid for the inducible bacterial expression of the bispecific scFv2(FRP5/225)-ETA (pMS238-5-225, pMS238-225-5, and pMS240-5-225)

11.1 Construction of the plasmid pMS238-5-225: pWW152-225 (1 µg) is digested with HindIII and SacI. DNA fragments are separated on a 1.0% (w/v) agarose gel (ultra pure agarose, BRL) and the expected 752 bp HindIII/SacI scFv(225) DNA fragment is eluted. pWW38 (50 ng) digested with HindIII and SacI and 30 ng of purified HindIII/SacI digested scFv(FRP5) DNA fragment are ligated. The resulting plasmid pWW38-225 is digested with SalI and AatII. A 1017 bp SalI/AatII fragment is purified by agarose gel electrophoresis and elution from the gel. pSW202-5 (50 ng) digested with SalI and AatII and 30 ng of purified 1017 bp SalI/AatII fragment are ligated and used to transform E.coli XL1 Blue (Stratagene). The following plasmid is obtained: pMS238-5-225. The nucleotide sequence encoding a scFv(225)-scFv(FRP5)-ETA fusion polypeptide and the corresponding amino acid sequence are shown in SEQ ID NO.13 and 14.
11.2 Construction of the plasmid pMS238-225-5: pSW202-225 (1 µg) is digested with NdeI and SalI. DNA fragments are separated on a 1.0% (w/v) agarose gel (ultra pure agarose, BRL) and the expected 1038 bp NdeI/SalI DNA fragment encoding the ompA signal peptide, the FLAG epitope, a cluster of 6 His residues, the scFv(225), a second cluster of 6 His residues and ETA sequences, is eluted. pMS238-5 vector fragment (50 ng) digested with NdeI and SalI and 30 ng of purified NdeI/SalI DNA fragment derived from pSW202-225 are ligated and used to transform E.coli XL1 Blue (Stratagene). The following plasmid is obtained: pMS238-225-5. The nucleotide sequence encoding a scFv(225)-scFv(FRP5)-ETA fusion polypeptide and the corresponding amino acid sequence are shown in SEQ ID NO.15 and 16.
11.3 Construction of the plasmid pMS240-5-225: pMS238-5-225 (1 µg) is completely digested with NdeI and partially digested with HindIII. DNA fragments are separated on a 1.0% (w/v) agarose gel (ultra pure agarose, BRL) and the expected 1218 bp NdeI/HindIII DNA fragment encoding the ompA signal peptide, the FLAG epitope, a cluster of six His residues, the scFv(FRP5), a second cluster of 6 His residues and ETA amino acids 252 to 366, is eluted. pSW202-225 (50 ng) digested with NdeI and HindIII and 30 ng of purified NdeI/HindIII DNA fragment derived from pMS238-5-225 are ligated and used to transform E.coli XLl Blue (Stratagene). The following plasmid is obtained: pMS240-5-225. The nucleotide sequence encoding a scFv(225)-scFv(FRP5)-ETA fusion polypeptide and the corresponding amino acid sequence are shown in SEQ ID NO.17 and 18.

### Example 12. Construction of plasmids for the inducible bacterial expression of bivalent scFv2(FRP5)-ETA proteins (pMS242-5-5 KDEL, pMS238-5-5, pMS246-5-5 KDEL)

12.1 Oligonucleotides: A double stranded DNA adaptor with XbaI and KpnI compatible ends is constructed by annealing 0.5 nmol of the oligonucleotide 5'-CTAGGCGAGAAAGATGAACTTTAATCTAGAGGTAC -3' with 0.5 nmol of the oligonucleotide 5'- CTCTAGATTAAAGTTCATCTTTCTCGC -3' by incubation at 65°C for 3 min and cooling to room temperature.
   Sequence of the adaptor: bp 1 to 4 XbaI compatible overhanging end; bp 32 to 35 KpnI compatible overhanging end.
12.2 Derivation of a scFv(FRP5) DNA fragment: pWW15-5 (Wels et al., Biotechnology 10: 1128, 1992) (1 µg) is completely digested with XbaI and partially digested with KpnI. DNA fragments are separated on a 1.0% (w/v) agarose gel (ultra pure agarose, BRL) and the expected XbaI/KpnI DNA fragment consisting of the vector backbone and the scFv(FRP5) encoding gene is eluted. XbaI/KpnI digested pWW15-5 (50 ng) and 20 pmol XbaI/KpnI oligonucleotide adaptor are ligated and used to transform E.coli XL1 Blue (Stratagene). The resulting plasmid is digested with HindIII and KpnI. DNA fragments are separated on a 1.0% (w/v) agarose gel (ultra pure agarose, BRL) and DNA of the expected size is eluted. pUCl9 (50 ng) digested with HindIII and KpnI and 30 ng of purified HindIII/KpnI digested scFv(FRP5) DNA fragment are ligated and used for transformation of E.coli XL1 Blue (Stratagene). The resulting plasmid is digested with HindIII and EcoRI. DNA fragments are separated on a 1.0% (w/v) agarose gel (ultra pure agarose, BRL) and a HindIII/EcoRI scFv(FRP5) DNA fragment of the expected size is eluted.
12.3 Polymerase chain reaction and derivation of an ETA DNA fragment: 25 ng of pWW20 (Wels et al., Cancer Res 52: 6310, 1992) is used for DNA amplification in a 50 µl reaction containing 50 pmol each of the two oligonucleotides complementary to regions in the ETA gene 5'-CTGCTGCGGGTCTATGTG -3' and 5'-GGCCGGTCGGTCGACGAATTCTTAAGCTTCGCGCGGCGGTTTGCC -3', 4 µl 2.5 mM dNTP (N= G, A, T, C) mixture, 5 µl 10x Taq DNA polymerase buffer (Boehringer Mannheim) and 2.5 U of Taq DNA polymerase (Boehringer Mannheim). Taq DNA polymerase is added after initial denaturation at 94°C for 2 min. For the first 5 cycles annealing is performed for 1 min at 42°C, for the following 25 cycles annealing is performed for 1 min at 55°C, primer extension for 1 min at 72°C, denaturation for 1 min at 94°C. Finally, amplification is completed by a 3 min primer extension step at 72°C. Amplification products are separated on a 1.2% (w/v) agarose gel (ultra pure agarose, BRL), DNA of the expected size is eluted using the QIAquick gel extraction kit (QIAGEN) according to procedures provided by the manufacturer, and subsequently digested with XhoI and SalI. The expected XhoI/SalI ETA DNA fragnent is separated on a 1.2% agarose gel and purified by elution from the gel as described above.
12.4 Construction of plasmid pWW220-5-5 KDEL: pWW215-5 (Wels et al., Cancer Res. 52: 6310, 1992) (50 ng) digested with XhoI and 30 ng of purified XhoI/SalI digested ETA amplification product are ligated and used to transform E.coli XL1 Blue (Stratagene). 50 ng of the resulting plasmid digested with HindIII and EcoRI and 30 ng of HindIII/EcoRI scFv(FRP5) DNA fragment are ligated and used for transformation of E.coli XL1 Blue (Stratagene). The following plasmid is obtained: pWW220-5-5 KDEL.
12.5 Construction of plasmid pMS201-5 KDEL: pSW200 (1 µg) is digested with NdeI and AatII. DNA fragments are separated on a 1.0% (w/v) agarose gel (ultra pure agarose, BRL) and the expected 597 bp NdeI/AatII DNA fragment is eluted. pWW220-5-5 KDEL is digested with NdeI and AatII. DNA fragments are separated and the expected NdeI/AatII vector DNA fragment is eluted. The 2 purified fragments are ligated and used to transform E.coli XL1 Blue (Stratagene). The following plasmid is obtained: pMS201-5 KDEL.
12.6 Construction of plasmid pMS242-5-5 KDEL: pMS238-5 (1 µg) is digested with SalI and AatII. DNA fragments are separated on a 1.0% (w/v) agarose gel (ultra pure agarose, BRL) and the expected 1017 bp SalI/AatII DNA fragment is eluted. pMS201-5 KDEL vector fragment digested with SalI and AatII (50 ng) and the 1017 bp SalI/AatII DNA fragment derived from pMS238-5 are ligated and used to transform E.coli XL1 Blue (Stratagene). The following plasmid is obtained: pMS242-5-5 KDEL. The nucleotide sequence encoding a scFv₂(FRP5)-ETA fusion polypeptide and the corresponding amino acid sequence are shown in SEQ ID NO.19 and 20.
12.7 Construction of plasmid pMS238-5-5: The 1017 bp SalI/AatII DNA fragment derived from pMS238-5 and pSW202-5 vector fragnent digested with SalI and AatII are ligated and used to transform E.coli XL1 Blue (Stratagene). The following plasmid is obtained: pMS238-5-5. The nucleotide sequence encoding a scFv₂(FRP5)-ETA fusion polypeptide and the corresponding amino acid sequence are shown in SEQ ID NO.21 and 22.
12.8 Construction of plasmid pMS246-5-5 KDEL: pSW202-5 (1 µg) is digested with NdeI and AatII. DNA fragments are separated on a 1.0% (w/v) agarose gel (ultra pure agarose, BRL) and the expected 1332 bp NdeI/AatII DNA fragment is eluted. pMS201-5 KDEL is digested with NdeI and AatII. DNA fragments are separated and the expected NdeI/AatII vector DNA fragment is eluted. The 2 purified fragments are ligated and used to transform E.coli XL1 Blue (Stratagene). The following plasmid is obtained: pMS246-5-5 KDEL. The nucleotide sequence encoding a scFv₂(FRP5)-ETA fusion polypeptide and the corresponding amino acid sequence are shown in SEQ ID No.23 and 24.

### Example 13. Bacterial expression and purification of monovalent and bivalent scFv-ETA and TGFα-ETA and scFv-TGFα-ETA proteins

Plasmids are transformed into E.coli CC118 (Manoil & Beckwith, Proc. Natl. Acad. Sci. USA 82: 8129, 1985). A recombinant single colony is grown overnight in 50 ml LB medium containing 100 µg/ml ampicillin and 0.6% glucose. The overnight culture is diluted 1:30 in 1 l fresh LB medium containing 100 µg/ml ampicillin and 0.6% glucose and grown at 37°C to an OD₅₅₀ of 0.5. IPTG is added to a final concentration of 0.5 mM and expression is induced for 45 min at room temperature. The cells are harvested at 4°C by centrifugation at 10,000 x g for 10 min in a J2-HS centrifuge (Beckman) using a JA10 rotor (Beckman).
13.1 Isolation of recombiant proteins from the bacterial cell pellet: The bacterial cell pellet is resuspended in 0.5 ml of lysis buffer containing 100 mM Tris-HCl, pH 8.0, 1 mg/ml lysozyme, 0.3 mM PMSF, 1 µg/ml aprotinin (Boehringer Mannheim), 0.5 µg/ml leupeptin (Boehringer Mannheim), and 1 µg/ml DNAse I. The bacterial cells are lysed by freezing in liquid nitrogen / thawing at 37°C. 15 ml PBS containing 6 M guanidine hydrochloride is added to the lysate. The suspension is gently shaken for 30 min at room temperature and then centrifuged at room temperature in a TL100 ultracentrifuge (Beckman) for 25 min at 100,000 x g. The supernatant is diluted to 3 M guanidine hydrochloride with PBS and stored at 4°C.
13.2 Purification of recombinant proteins by affinity chromatography: A column is packed with 3 ml chelating sepharose (Pharmacia). 5 ml of 2% NiSO₄ solution are passed over the column followed by washing with 5 ml water and 6 ml 150 mM NaCl, 50 mM acetic acid, pH 4.0. The column is neutralized with 20 ml PBS followed by equilibration with 7 ml 3 M guanidine hydrochloride, 20 mM imidazole in PBS. The supernatant of the bacterial lysate is adjusted to 10 mM imidazole and passed through the column. The column is washed with 25 ml 3 M guanidine hydrochloride, 20 mM imidazole in PBS followed by 9 ml 3 M guanidine hydrochloride, 50 mM imidazole in PBS. Elution of specifically bound proteins is carried out with 3 M guanidine hydrochloride, 250 mM imidazole in PBS. Protein containing samples are pooled and dialysed twice for 6 h at 4°C against PBS. The dialysed material is clarified by centrifugation at 4°C in a TL100 ultracentrifuge (Beckman) for 25 min at 100,000 x g. Typical yield of purified proteins is 1 mg to 2 mg per 1 of original bacterial culture. Typical purity of obtained materials is greater than 70% determined by SDS-PAGE and Coomassie brilliant blue staining. The following proteins are obtained following the procedure described above: scFv(225)-ETA (plasmid pSW202-225), scFv(FRP5)-ETA (plasmid pSW202-5), scFv2(FRP5/225)-ETA (plasmid pMS240-5-225), TGFα-ETA (pSW202-TGF), scFv(FRP5)/TGFα-ETA (plasmid pMS238-5-TGF).

### Example 14. Determination of cell killing activity

The specificity and in vitro cell killing activity of the recombinant purified proteins described in Example 13 is determined in cell viability experiments using the human breast carcinoma cell lines SK- BR-3 (ATCC HTB 30), MDA-MB-468 (ATCC HTB 132), and the human epidermoid squamous cell carcinoma cell line A431 (ATCC CRL 1555). MDA-MB-468 cells express approximately 1.5 x 10⁶ EGF receptors per cell and no detectable erbB-2 protein. SK-BR-3 cells express approximately 9 x 10⁴ EGF receptors and 1 x 10⁶ erbB-2 receptors per cell. A431 cells express approximately 2 x 10⁶ EGF receptors and 2 x 10⁵ erbB-2 receptors per cell.
14.1 Cell viability assay: SK-BR-3, MDA-MB-468 and A431 cells are grown in 96-well tissue culture plates (Falcon) at a density of 1 x 10⁴ cells per well in 100 µl DMEM containing 8% heat inactivated FCS in the presence of 0, 1, 10, 100, and 1000 ng/ml of the recombinant toxin fusion proteins scFv(225)-ETA, scFv(FRP5)-ETA, scFv2(FRP5/225)-ETA, TGFα-ETA, or scFv(FRP5)/TGFα-ETA at 37°C in a humid atmosphere containing 5% CO₂ for 40 hours. 10 µl of 10 mg/ml MTT (3-(4,5- dimethylthiazole-2-yl)-2,5 diphenyltetrazolium bromide) (Sigma) in PBS are added to each well and the cells are incubated for another 3 h. 90 µl of lysis buffer containing 20% SDS in 50% dimethyl formamide, pH 4.7 are added to each well followed by incubation for 3 h at 37°C. The relative amount of viable cells in each sample is determined as the OD at 590 nm of the lysate in a microplate reader (Dynatech). A sample containing 100 µl DMEM, 10 µl MTT, and 90 µl lysis buffer is used as blank. Cells grown without the addition of recombinant proteins are set as 100% viability. The following molar concentrations (50% inhibiting concentration, IC50) are determined to reduce the relative amount of viable cells by 50%:

| Cell line | IC50 (nM) | | |
|---|---|---|---|
| | SK-BR-3 | A431 | MDA-MB-468 |
| scFv(225)-ETA | >20 | 0.07 | 2.27 |
| scFv(FRP5)-ETA | 0.34 | 0.52 | >20 |
| scFv2(FRP5/225)-ETA | 0.32 | 0.02 | 3.04 |
| TGFα-ETA | 1.53 | 0.15 | 0.11 |
| scFv(FRP5)/TGFα-ETA | 0.53 | 0.06 | 0.92 |

14.2 Competition of cytotoxicity with monoclonal antibodies 225 and FRP5: A431 cells are used in an in vitro cell viability assay as described above. The cells are grown for 40 h in the presence of 100 ng/ml scFv2(FRP5/225)-ETA or scFv(FRP5)/TGFα-ETA in the absence or presence of a 160-fold excess of monoclonal antibody FRP5, 225, or both as competitors. The monoclonal antibody 225 inhibits the binding of EGF and TGF-α to the human EGF receptor (Sunada et al., Proc. Natl. Acad. Sci. USA 83: 3825, 1986). The relative amount of viable cells (cell viability) after treatment is determined as described above. The following results are obtained:

| Treatment of A431 cells | Cell viability (% of control) |
|---|---|
| scFv2(FRP5/225)-ETA | 8.7 |
| scFv2(FRP5/225)-ETA + mAb 225 | 45.8 |
| scFv2(FRP5/225)-ETA + mAb FRP5 | 10.4 |
| scFv2(FRP5/225)-ETA + mAbs 225 and FRP5 | 67.7 |
| scFv(FRP5)/TGFα-ETA | 10.7 |
| scFv(FRP5)/TGFα-ETA + mAb 225 | 36.9 |
| scFv(FRP5)/TGFα-ETA + mAb FRP5 | 18.0 |
| scFv(FRP5)/TGFα-ETA + mAbs 225 and FRP5 | 68.0 |

MAb FRP5 inhibits the binding of the scFv(FRP5) antibody domain to the erbB-2 receptor and mAb 225 inhibits the binding of the scFv(225) antibody domain and the TGF-α domain to the EGF receptor leading to a reduction of the cytotoxic effect of the bispecific recombinant scFv2(FRP5/225)-ETA and scFv(FRP5)/TGFα-ETA proteins. Combination of both competing mAbs leads to an additional reduction of the cytotoxic effect showing that both binding domains in each recombinant fusion protein are active and contribute to the delivery of the toxin molecules into the A431 target cells.

### Example 15. Activation of the EGF receptor and erbB-2 receptor tyrosine kinases

15.1 Stimulation of A431 cells with recombinant proteins:
   A431 cells are grown for 16 h at 37°C at a density of 1 x 10⁶ cells per well in 6 well plates (Falcon) in DMEM containing 0.5% heat inactivated FCS in a humid atmosphere containing 5% CO₂. Purified recombinant proteins scFv(225)-ETA, scFv(FRP5)-ETA, scFv2(FRP5/225)-ETA, TGFα-ETA, or scFv(FRP5)/TGFα-ETA are added at a concentration of 10 µg/ml followed by incubation for 10 min at 37°C. Control cells are treated with PBS or 100 ng/ml EGF. Cells are placed on ice and washed with 3 ml per well PBS containing 200 M Na-V0₄. Cells are lysed for 10 min at 4°C with 500 µl lysis buffer per well containing 50 mM Tris-HCl, pH7.5; 5 mM EGTA, pH 8.5; 150 mM NaCl; 18 Triton X-100; 10 µg/ml aprotinin; 10 µg/ml leupeptin; 1 mM PMSF; 2 mM Na-VO₄; 50 mM NaF; 10 mM Na-molybdate. Lysates are clarified by centrifugation for 10 min at 4°C at 12,000 x g.
15.2 Analysis of phosphotyrosine contents of EGF receptors in A431 cells: Cleared cell lysates from 15.1 containing 15 µg each of total proteins are applied on a 7.5% SDS-PAGE. After electrophoresis, proteins are blotted for 1 h 25 min at 0.8 mA per cm² on a PVDF membrane (Millipore) Non-specifc binding of antibodies to the membrane is blocked by incubation in blocking buffer containing TTBS (10 mM Tris-HCl, pH7 5, 150 mM NaCl, 0.05% Tween 20) and 3% skim milk powder for 1 h at room temperature. Phosphotyrosine containing proteins are detected by incubation of the membrane with an anti-phosphotyrosine monoclonal antibody in blocking buffer as described by Harwerth et al. (J. Biol. Chem. 267: 15160, 1992) followed by incubation with an anti-mouse horse radish peroxidase coupled antibody and chemiluminescent detection with the ECL kit (Amersham) according to the manufacturer's protocol. A strong increase of EGF receptor phosphotyrosine content as an indicator for receptor activation in comparison to PBS treated control cells is observed in A431 cells treated with EGF, scFv2(FRP5/225)-ETA, TGFα-ETA, or scFv(FRP5)/TGFα-ETA, but not in A431 cells treated with scFv(225)-ETA, or scFv(FRP5)- ETA. The residual phosphotyrosine content of EGF receptors observed in A431 cells treated with PBS as a control or scFv(FRP5) is completely abrogated in cells treated with scFv(225)-ETA.
15.3 Analysis of phosphotyrosine contents of erbB-2 receptors in A431 cells: 400 µl each of clarified cell lysates from 15.1 are incubated with a polyclonal rabbit anti-erbB-2 antibody for 1 h on ice. 50 µl of a protein A sepharose slurry (Pharmacia) are added to each sample. Samples are incubated for 45 min at 4°C with constant agitating to allow the formation of complexes. The protein A sepharose beads are washed twice with 1 ml of a buffer containing 50 mM Tris-HCl, pH 7.5; 140 mM NaCl; 5mM EDTA; 1% Triton X-100 followed by washing once with 1 ml of a buffer containing 50 mM Tris-HCl, pH 7.5; 140 mM NaCl; 5 mM NaCl. Protein A sepharose bound proteins are released by boiling in 40 µl 2 x loading buffer containing 5% 2-mercaptoethanol. 20 µl each of the solutions are applied to a 7.5% SDS-PAGE followed by immunoblotting and detection of phosphotyrosine containing proteins with an anti- phosphotyrosine monoclonal antibody as described above. An increase of erbB-2 receptor phosphotyrosine content as an indicator for receptor activation in comparison to PBS treated control cells is observed in A431 cells treated with EGF, scFv2(FRP5/225)-ETA, TGFα-ETA, or scFv(FRP5)/TGFα-ETA, but not in A431 cells treated with scFv(225)-ETA or scFv(FRP5)-ETA.

SEQ ID NO: 1 and 2
SEQUENCE TYPE: nucleotide
SEQUENCE LENGTH: 794 bp
MOLECULE TYPE: plasmid DNA
ORIGINAL SOURCE ORGANISM: mouse; synthetic
IMMEDIATE EXPERIMENTAL SOURCE: E.coli
NAME OF CELL CLONE: pWW152-225

FEATURES: from 43 to 399 bp monoclonal antibody 225 VH cDNA
from 400 to 444 bp synthetic spacer
from 445 to 762 bp monoclonal antibody 225 VL cDNA

PROPERTIES: encodes scFv(225)

10 30 50
GGTACCGGGCCCCCCCTCGAGGTCGACGGTATCGATAAGCTTCAGGTACAACTGCAGGAG
GlnValGlnLeuGlnGlu
70 90 110
TCAGGACCTGGCCTAGTGCAGCCCTCACAGAGCCTGTCCATCACCTGCACAGTCTCTGGT
SerGlyProGlyLeuValGlnProSerGlnSerLeuSerIleThrCysThrValSerGly
130 150 170
TTCTCATTAACTAACTATGGTGTACACTGGGTTCGCCAGTCTCCAGGAAAGGGTCTGGAG
PheSerLeuThrAsnTyrGlyValHisTrpValArgGlnSerProGlyLysGlyLeuGlu
190 210 230
TGGCTGGGAGTGATATGGAGTGGTGGAAACACAGACTATAATACACCTTTCACATCCAGA
TrpLeuGlyValIleTrpSerGlyGlyAsnThrAspTyrAsnThrProPheThrSerArg
250 270 290
CTGAGCATCAACAAGGACAATTCCAAGAGCCAAGTTTTCTTTAAAATGAACAGTCTGCAA
LeuSerIleAsnLysAspAsnSerLysSerGlnValPhePheLysMetAsnSerLeuGln
310 330 350
TCTAATGACACAGCCATATATTACTGTGCCAGAGCCCTCACCTACTATGATTACGAGTTT
SerAsnAspThrAlaIleTyrTyrCysAlaArgAlaLeuThrTyrTyrAspTyrGluPhe
370 390 410
GCTTACTGGGGCCAAGGGACCACGGTCACCGTTTCCTCTGGCGGTGGCGGTTCTGGTGGC
AlaTyrTrpGlyGlnGlyThrThrValThrValSerSerGlyGlyGlyGlySerGlyGly
430 450 470
GGTGGCTCCGGCGGTGGCGGTTCTGACATCCAGCTGACCCAGTCTCCAGTCATCCTGTCT
GlyGlySerGlyGlyGlyGlySerAspIleGlnLeuThrGlnSerProValIleLeuSer
490 510 530
GTGAGTCCAGGAGAAAGAGTCAGTTTCTCCTGCAGGGCCAGTCAGAGTATTGGCACAAAC
ValSerProGlyGluArgValSerPheSerCysArgAlaSerGlnSerIleGlyThrAsn
550 570 590
ATACACTGGTATCAGCAAAGAACAAATGGTTCTCCAAGGCTTCTCATAAAGTATGCTTCT
IleHisTrpTyrGlnGlnArgThrAsnGlySerProArgLeuLeuIleLysTyrAlaSer
610 630 650
GAGTCTATCTCTGGGATCCCTTCCAGGTTTAGTGGCAGTGGATCAGGGACAGATTTTACT
GluSerIleSerGlyIleProSerArgPheSerGlySerGlySerGlyThrAspPheThr
670 690 710
CTTAGCATCAACAGTGTGGAGTCTGAAGATATTGCAGATTATTATTGTCAACAAAATAAT
LeuSerIleAsnSerValGluSerGluAspIleAlaAspTyrTyrCysGlnGlnAsnAsn
730 750 770
AACTGGCCAACCACGTTCGGTGCTGGGACCAAGCTGGAGATCAAAGCTCTAGAGGATCCC
AsnTrpProThrThrPheGlyAlaGlyThrLysLeuGluIle
790
CGGGTACCGAGCTC

SEQ ID NO: 3 and 4
SEQUENCE TYPE: nucleotide
SEQUENCE LENGTH: 794 bp
MOLECULE TYPE: plasmid DNA
ORIGINAL SOURCE ORGANISM: mouse; synthetic
IMMEDIATE EXPERIMENTAL SOURCE: E.coli
NAME OF CELL CLONE: pWW152-5

FEATURES: from 43 to 399 bp monoclonal antibody FRP5 VH cDNA
from 400 to 444 bp synthetic spacer
from 445 to 762 bp monoclonal antibody FRP5 VL cDNA

PROPERTIES: encodes scFv(FRP5)

10 30 50
GGTACCGGGCCCCCCCTCGAGGTCGACGGTATCGATAAGCTTCAGGTACAACTGCAGCAG
GlnValGlnLeuGlnGln
70 90 110
TCTGGACCTGAACTGAAGAAGCCTGGAGAGACAGTCAAGATCTCCTGCAAGGCCTCTGGG
SerGlyProGluLeuLysLysProGlyGluThrValLysIleSerCysLysAlaSerGly
130 150 170
TATCCTTTCACAAACTATGGAATGAACTGGGTGAAGCAGGCTCCAGGACAGGGTTTAAAG
TyrProPheThrAsnTyrGlyMetAsnTrpValLysGlnAlaProGlyGlnGlyLeuLys
190 210 230
TGGATGGGCTGGATTAACACCTCCACTGGAGAGTCAACATTTGCTGATGACTTCAAGGGA
TrpMetGlyTrpIleAsnThrSerThrGlyGluSerThrPheAlaAspAspPheLysGly
250 270 290
CGGTTTGACTTCTCTTTGGAAACCTCTGCCAACACTGCCTATTTGCAGATCAACAACCTC
ArgPheAspPheSerLeuGluThrSerAlaAsnThrAlaTyrLeuGlnIleAsnAsnLeu
310 330 350
AAAAGTGAAGACATGGCTACATATTTCTGTGCAAGATGGGAGGTTTACCACGGCTACGTT
LysSerGluAspMetAlaThrTyrPheCysAlaArgTrpGluValTyrHisGlyTyrVal
370 390 410
CCTTACTGGGGCCAAGGGACCACGGTCACCGTTTCCTCTGGCGGTGGCGGTTCTGGTGGC
ProTyrTrpGlyGlnGlyThrThrValThrValSerSerGlyGlyGlyGlySerGlyGly
430 450 470
GGTGGCTCCGGCGGTGGCGGTTCTGACATCCAGCTGACCCAGTCTCACAAATTCCTGTCC
GlyGlySerGlyGlyGlyGlySerAspIleGlnLeuThrGlnSerHisLysPheLeuSer
490 510 530
ACTTCAGTAGGAGACAGGGTCAGCATCACCTGCAAGGCCAGTCAGGATGTGTATAATGCT
ThrSerValGlyAspArgValSerIleThrCysLysAlaSerGlnAspValTyrAsnAla
550 570 590
GTTGCCTGGTATCAACAGAAACCAGGACAATCTCCTAAACTTCTGATTTACTCGGCATCC
ValAlaTrpTyrGlnGlnLysProGlyGlnSerProLysLeuLeuIleTyrSerAlaSer
610 630 650
TCCCGGTACACTGGAGTCCCTTCTCGCTTCACTGGCAGTGGCTCTGGGCCGGATTTCACT
SerArgTyrThrGlyValProSerArgPheThrGlySerGlySerGlyProAspPheThr
670 690 710
TTCACCATCAGCAGTGTGCAGGCTGAAGACCTGGCAGTTTATTTCTGTCAGCAACATTTT
PheThrIleSerSerValGlnAlaGluAspLeuAlaValTyrPheCysGlnGlnHisPhe
730 750 770
CGTACTCCATTCACGTTCGGCTCGGGGACAAAATTGGAGATCAAAGCTCTAGAGGATCCC
ArgThrProPheThrPheGlySerGlyThrLysLeuGluIle
790
CGGGTACCGAGCTC

SEQ ID NO: 5 and 6
SEQUENCE TYPE: nucleotide
SEQUENCE LENGTH: 2070 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pSW202-225

FEATURES: from 1 to 63 bp ompA signal peptide
from 64 to 114 bp synthetic spacer
from 115 to 834 bp scFv(225) cDNA
from 835 to 867 bp synthetic spacer
from 868 to 1956 bp ETA gene amino acids 252 to 613

PROPERTIES: encodes scFv(225)-ETA

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGGAGTCAGGACCTGGCCTAGTGCAGCCCTCACAGAGCCTGTCCATCACCTGC
GlnLeuGlnGluSerGlyProGlyLeuValGlnProSerGlnSerLeuSerIleThrCys
190 210 230
ACAGTCTCTGGTTTCTCATTAACTAACTATGGTGTACACTGGGTTCGCCAGTCTCCAGGA
ThrValSerGlyPheSerLeuThrAsnTyrGlyValHisTrpValArgGlnSerProGly
250 270 290
AAGGGTCTGGAGTGGCTGGGAGTGATATGGAGTGGTGGAAACACAGACTATAATACACCT
LysGlyLeuGluTrpLeuGlyValIleTrpSerGlyGlyAsnThrAspTyrAsnThrPro
310 330 350
TTCACATCCAGACTGAGCATCAACAAGGACAATTCCAAGAGCCAAGTTTTCTTTAAAATG
PheThrSerArgLeuSerIleAsnLysAspAsnSerLysSerGlnValPhePheLysMet
370 390 410
AACAGTCTGCAATCTAATGACACAGCCATATATTACTGTGCCAGAGCCCTCACCTACTAT
AsnSerLeuGlnSerAsnAspThrAlaIleTyrTyrCysAlaArgAlaLeuThrTyrTyr
430 450 470
GATTACGAGTTTGCTTACTGGGGCCAAGGGACCACGGTCACCGTTTCCTCTGGCGGTGGC
AspTyrGluPheAlaTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCCA
GlySerGlyGlyGlyGlySerGlyGlyGlyGlySerAspIleGlnLeuThrGlnSerPro
550 570 590
GTCATCCTGTCTGTGAGTCCAGGAGAAAGAGTCAGTTTCTCCTGCAGGGCCAGTCAGAGT
ValIleLeuSerValSerProGlyGluArgValSerPheSerCysArgAlaSerGlnSer
610 630 650
ATTGGCACAAACATACACTGGTATCAGCAAAGAACAAATGGTTCTCCAAGGCTTCTCATA
IleGlyThrAsnIleHisTrpTyrGlnGlnArgThrAsnGlySerProArgLeuLeuIle
670 690 710
AAGTATGCTTCTGAGTCTATCTCTGGGATCCCTTCCAGGTTTAGTGGCAGTGGATCAGGG
LysTyrAlaSerGluSerIleSerGlyIleProSerArgPheSerGlySerGlySerGly
730 750 770
ACAGATTTTACTCTTAGCATCAACAGTGTGGAGTCTGAAGATATTGCAGATTATTATTGT
ThrAspPheThrLeuSerIleAsnSerValGluSerGluAspIleAlaAspTyrTyrCys
790 810 830
CAACAAAATAATAACTGGCCAACCACGTTCGGTGCTGGGACCAAGCTGGAGATCAAAGCT
GlnGlnAsnAsnAsnTrpProThrThrPheGlyAlaGlyThrLysLeuGluIleLysAla
850 870 890
CTAGAgcaccatcatcaccatcacctAGAGGGCGGCAGCCTGGCCGCGCTGACCGCGCAC
LeuGluHisHisHisHisHisHisLeuGluGlyGlySerLeuAlaAlaLeuThrAlaHis

910 930 950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970 990 1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030 1050 1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090 1110 1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150 1170 1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210 1230 1250
GCCAACGCCGACGTGGTGAGCCTGACCTGCCCGGTCGCCGCCGGTGAATGCGCGGGCCCG
AlaAsnAlaAspValValSerLeuThrCysProValAlaAlaGlyGluCysAlaGlyPro
1270 1290 1310
GCGGACAGCGGCGACGCCCTGCTGGAGCGCAACTATCCCACTGGCGCGGAGTTCCTCGGC
AlaAspSerGlyAspAlaLeuLeuGluArgAsnTyrProThrGlyAlaGluPheLeuGly
1330 1350 1370
GACGGCGGCGACGTCAGCTTCAGCACCCGCGGCACGCAGAACTGGACGGTGGAGCGGCTG
AspGlyGlyAspValSerPheSerThrArgGlyThrGlnAsnTrpThrValGluArgLeu
1390 1410 1430
CTCCAGGCGCACCGCCAACTGGAGGAGCGCGGCTATGTGTTCGTCGGCTACCACGGCACC
LeuGlnAlaHisArgGlnLeuGluGluArgGlyTyrValPheValGlyTyrHisGlyThr
1450 1470 1490
TTCCTCGAAGCGGCGCAAAGCATCGTCTTCGGCGGGGTGCGCGCGCGCAGCCAGGACCTC
PheLeuGluAlaAlaGlnSerIleValPheGlyGlyValArgAlaArgSerGlnAspLeu
1510 1530 1550
GACGCGATCTGGCGCGGTTTCTATATCGCCGGCGATCCGGCGCTGGCCTACGGCTACGCC
AspAlaIleTrpArgGlyPheTyrIleAlaGlyAspProAlaLeuAlaTyrGlyTyrAla
1570 1590 1610
CAGGACCAGGAACCCGACGCACGCGGCCGGATCCGCAACGGTGCCCTGCTGCGGGTCTAT
GlnAspGlnGluProAspAlaArgGlyArgIleArgAsnGlyAlaLeuLeuArgValTyr
1630 1650 1670
GTGCCGCGCTCGAGCCTGCCGGGCTTCTACCGCACCAGCCTGACCCTGGCCGCGCCGGAG
ValProArgSerSerLeuProGlyPheTyrArgThrSerLeuThrLeuAlaAlaProGlu
1690 1710 1730
GCGGCGGGCGAGGTCGAACGGCTGATCGGCCATCCGCTGCCGCTGCGCCTGGACGCCATC
AlaAlaGlyGluValGluArgLeuIleGlyHisProLeuProLeuArgLeuAspAlaIle
1750 1770 1790
ACCGGCCCCGAGGAGGAAGGCGGGCGCCTGGAGACCATTCTCGGCTGGCCGCTGGCCGAG
ThrGlyProGluGluGluGlyGlyArgLeuGluThrIleLeuGlyTrpProLeuAlaGlu
1810 1830 1850
CGCACCGTGGTGATTCCCTCGGCGATCCCCACCGACCCGCGCAACGTCGGCGGCGACCTC
ArgThrValValIleProSerAlaIleProThrAspProArgAsnValGlyGlyAspLeu
1870 1890 1910
GACCCGTCCAGCATCCCCGACAAGGAACAGGCGATCAGCGCCCTGCCGGACTACGCCAGC
AspProSerSerIleProAspLysGluGlnAlaIleSerAlaLeuProAspTyrAlaSer
1930 1950 1970
CAGCCCGGCAAACCGCCGCGCGAGGACCTGAAGTAACTGCCGCGACCGGCCGGCTCCCTT
GlnProGlyLysProProArgGluAspLeuLysEnd
1990 2010 2030
CGCAGGAGCCGGCCTTCTCGGGGCCTGGCCATACATCAGGTTTTCCTGATGCCAGCCCAA
2050 2070
TCGAATATGAATTTCGACAGATCTCTCGAG

SEQ ID NO: 7 and 8
SEQUENCE TYPE: nucleotide
SEQUENCE LENGTH: 2070 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pSW202-5

FEATURES: from 1 to 63 bp ompA signal peptide
from 64 to 114 bp synthetic spacer
from 115 to 834 bp scFv(FRP5) cDNA
from 835 to 867 bp synthetic spacer
from 868 to 1956 bp ETA gene amino acids 252 to 613

PROPERTIES: encodes scFv(FRP5)-ETA

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGCAGTCTGGACCTGAACTGAAGAAGCCTGGAGAGACAGTCAAGATCTCCTGC
GlnLeuGlnGlnSerGlyProGluLeuLysLysProGlyGluThrValLysIleSerCys
190 210 230
AAGGCCTCTGGGTATCCTTTCACAAACTATGGAATGAACTGGGTGAAGCAGGCTCCAGGA
LysAlaSerGlyTyrProPheThrAsnTyrGlyMetAsnTrpValLysGlnAlaProGly
250 270 290
CAGGGTTTAAAGTGGATGGGCTGGATTAACACCTCCACTGGAGAGTCAACATTTGCTGAT
GlnGlyLeuLysTrpMetGlyTrpIleAsnThrSerThrGlyGluSerThrPheAlaAsp
310 330 350
GACTTCAAGGGACGGTTTGACTTCTCTTTGGAAACCTCTGCCAACACTGCCTATTTGCAG
AspPheLysGlyArgPheAspPheSerLeuGluThrSerAlaAsnThrAlaTyrLeuGln
370 390 410
ATCAACAACCTCAAAAGTGAAGACATGGCTACATATTTCTGTGCAAGATGGGAGGTTTAC
IleAsnAsnLeuLysSerGluAspMetAlaThrTyrPheCysAlaArgTrpGluValTyr
430 450 470
CACGGCTACGTTCCTTACTGGGGCCAAGGGACCACGGTCACCGTTTCCTCTGGCGGTGGC
HisGlyTyrValProTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCAC
GlySerGlyGlyGlyGlySerGlyGlyGlyGlySerAspIleGlnLeuThrGlnSerHis
550 570 590
AAATTCCTGTCCACTTCAGTAGGAGACAGGGTCAGCATCACCTGCAAGGCCAGTCAGGAT
LysPheLeuSerThrSerValGlyAspArgValSerIleThrCysLysAlaSerGlnAsp
610 630 650
GTGTATAATGCTGTTGCCTGGTATCAACAGAAACCAGGACAATCTCCTAAACTTCTGATT
ValTyrAsnAlaValAlaTrpTyrGlnGlnLysProGlyGlnSerProLysLeuLeuIle
670 690 710
TACTCGGCATCCTCCCGGTACACTGGAGTCCCTTCTCGCTTCACTGGCAGTGGCTCTGGG
TyrSerAlaSerSerArgTyrThrGlyValProSerArgPheThrGlySerGlySerGly
730 750 770
CCGGATTTCACTTTCACCATCAGCAGTGTGCAGGCTGAAGACCTGGCAGTTTATTTCTGT
ProAspPheThrPheThrIleSerSerValGlnAlaGluAspLeuAlaValTyrPheCys
790 810 830
CAGCAACATTTTCGTACTCCATTCACGTTCGGCTCGGGGACAAAATTGGAGATCAAAGCT
GlnGlnHisPheArgThrProPheThrPheGlySerGlyThrLysLeuGluIleLysAla
850 870 890
CTAGAgcaccatcatcaccatcacctAGAGGGCGGCAGCCTGGCCGCGCTGACCGCGCAC
LeuGluHisHisHisHisHisHisLeuGluGlyGlySerLeuAlaAlaLeuThrAlaHis

910 930 950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970 990 1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030 1050 1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090 1110 1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150 1170 1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210 1230 1250
GCCAACGCCGACGTGGTGAGCCTGACCTGCCCGGTCGCCGCCGGTGAATGCGCGGGCCCG
AlaAsnAlaAspValValSerLeuThrCysProValAlaAlaGlyGluCysAlaGlyPro
1270 1290 1310
GCGGACAGCGGCGACGCCCTGCTGGAGCGCAACTATCCCACTGGCGCGGAGTTCCTCGGC
AlaAspSerGlyAspAlaLeuLeuGluArgAsnTyrProThrGlyAlaGluPheLeuGly
1330 1350 1370
GACGGCGGCGACGTCAGCTTCAGCACCCGCGGCACGCAGAACTGGACGGTGGAGCGGCTG
AspGlyGlyAspValSerPheSerThrArgGlyThrGlnAsnTrpThrValGluArgLeu
1390 1410 1430
CTCCAGGCGCACCGCCAACTGGAGGAGCGCGGCTATGTGTTCGTCGGCTACCACGGCACC
LeuGlnAlaHisArgGlnLeuGluGluArgGlyTyrValPheValGlyTyrHisGlyThr
1450 1470 1490
TTCCTCGAAGCGGCGCAAAGCATCGTCTTCGGCGGGGTGCGCGCGCGCAGCCAGGACCTC
PheLeuGluAlaAlaGlnSerIleValPheGlyGlyValArgAlaArgSerGlnAspLeu
1510 1530 1550
GACGCGATCTGGCGCGGTTTCTATATCGCCGGCGATCCGGCGCTGGCCTACGGCTACGCC
AspAlaIleTrpArgGlyPheTyrIleAlaGlyAspProAlaLeuAlaTyrGlyTyrAla
1570 1590 1610
CAGGACCAGGAACCCGACGCACGCGGCCGGATCCGCAACGGTGCCCTGCTGCGGGTCTAT
GlnAspGlnGluProAspAlaArgGlyArgIleArgAsnGlyAlaLeuLeuArgValTyr
1630 1650 1670
GTGCCGCGCTCGAGCCTGCCGGGCTTCTACCGCACCAGCCTGACCCTGGCCGCGCCGGAG
ValProArgSerSerLeuProGlyPheTyrArgThrSerLeuThrLeuAlaAlaProGlu
1690 1710 1730
GCGGCGGGCGAGGTCGAACGGCTGATCGGCCATCCGCTGCCGCTGCGCCTGGACGCCATC
AlaAlaGlyGluValGluArgLeuIleGlyHisProLeuProLeuArgLeuAspAlaIle
1750 1770 1790
ACCGGCCCCGAGGAGGAAGGCGGGCGCCTGGAGACCATTCTCGGCTGGCCGCTGGCCGAG
ThrGlyProGluGluGluGlyGlyArgLeuGluThrIleLeuGlyTrpProLeuAlaGlu
1810 1830 1850
CGCACCGTGGTGATTCCCTCGGCGATCCCCACCGACCCGCGCAACGTCGGCGGCGACCTC
ArgThrValValIleProSerAlaIleProThrAspProArgAsnValGlyGlyAspLeu
1870 1890 1910
GACCCGTCCAGCATCCCCGACAAGGAACAGGCGATCAGCGCCCTGCCGGACTACGCCAGC
AspProSerSerIleProAspLysGluGlnAlaIleSerAlaLeuProAspTyrAlaSer
1930 1950 1970
CAGCCCGGCAAACCGCCGCGCGAGGACCTGAAGTAACTGCCGCGACCGGCCGGCTCCCTT
GlnProGlyLysProProArgGluAspLeuLysEnd
1990 2010 2030
CGCAGGAGCCGGCCTTCTCGGGGCCTGGCCATACATCAGGTTTTCCTGATGCCAGCCCAA
2050 2070
TCGAATATGAATTTCGACAGATCTCTCGAG

SEQ ID NO: 9 and 10
SEQUENCE TYPE: nucleotide
SEQUENCE LENGTH: 1506 bp
MOLECULE TYPE: plasmid DNA
ORIGINAL SOURCE ORGANISM: human; P. aeruginosa; synthetic
IMMEDIATE EXPERIMENTAL SOURCE: E.coli
NAME OF CELL CLONE: pSW202-TGF

FEATURES: from 1 to 63 bp ompA signal peptide
from 64 to 123 bp synthetic spacer
from 124 to 273 bp human TGF-α cDNA
from 274 to 303 bp synthetic spacer
from 304 to 1392 bp ETA gene amino acids 252 to 613

PROPERTIES: encodes TGFα-ETA

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTGGTACC
AlaAspTyrLysAspAspAspAspLysLeuHisHisHisHisHisHisLysLeuGlyThr
130 150 170
GGTGTGGTGTCCCATTTTAATGACTGCCCAGATTCCCACACTCAGTTCTGCTTCCATGGA
GlyValValSerHisPheAsnAspCysProAspSerHisThrGlnPheCysPheHisGly
190 210 230
ACCTGCAGGTTTTTGGTGCAGGAGGACAAGCCAGCATGTGTCTGCCATTCTGGGTACGTT
ThrCysArgPheLeuValGlnGluAspLysProAlaCysValCysHisSerGlyTyrVal
250 270 290
GGTGCACGCTGTGAGCATGCGGACCTCCTGGCCTCTCTAGAGCACCATCATCACCATCAC
GlyAlaArgCysGluHisAlaAspLeuLeuAlaSerLeuGluHisHisHisHisHisHis
310 330 350
CTAGAGGGCGGCAGCCTGGCCGCGCTGACCGCGCACCAGGCCTGCCACCTGCCGCTGGAG
LeuGluGlyGlySerLeuAlaAlaLeuThrAlaHisGlnAlaCysHisLeuProLeuGlu
370 390 410
ACTTTCACCCGTCATCGCCAGCCGCGCGGCTGGGAACAACTGGAGCAGTGCGGCTATCCG
ThrPheThrArgHisArgGlnProArgGlyTrpGluGlnLeuGluGlnCysGlyTyrPro
430 450 470
GTGCAGCGGCTGGTCGCCCTCTACCTGGCGGCGCGACTGTCATGGAACCAGGTCGACCAG
ValGlnArgLeuValAlaLeuTyrLeuAlaAlaArgLeuSerTrpAsnGlnValAspGln
490 510 530
GTGATCCGCAACGCCCTGGCCAGCCCCGGCAGCGGCGGCGACCTGGGCGAAGCGATCCGC
ValIleArgAsnAlaLeuAlaSerProGlySerGlyGlyAspLeuGlyGluAlaIleArg
550 570 590
GAGCAGCCGGAGCAGGCCCGTCTGGCCCTGACCCTGGCCGCCGCCGAGAGCGAGCGCTTC
GluGlnProGluGlnAlaArgLeuAlaLeuThrLeuAlaAlaAlaGluSerGluArgPhe
610 630 650
GTCCGGCAGGGCACCGGCAACGACGAGGCCGGCGCGGCCAACGCCGACGTGGTGAGCCTG
ValArgGlnGlyThrGlyAsnAspGluAlaGlyAlaAlaAsnAlaAspValValSerLeu
670 690 710
ACCTGCCCGGTCGCCGCCGGTGAATGCGCGGGCCCGGCGGACAGCGGCGACGCCCTGCTG
ThrCysProValAlaAlaGlyGluCysAlaGlyProAlaAspSerGlyAspAlaLeuLeu
730 750 770
GAGCGCAACTATCCCACTGGCGCGGAGTTCCTCGGCGACGGCGGCGACGTCAGCTTCAGC
GluArgAsnTyrProThrGlyAlaGluPheLeuGlyAspGlyGlyAspValSerPheSer
790 810 830
ACCCGCGGCACGCAGAACTGGACGGTGGAGCGGCTGCTCCAGGCGCACCGCCAACTGGAG
ThrArgGlyThrGlnAsnTrpThrValGluArgLeuLeuGlnAlaHisArgGlnLeuGlu
850 870 890
GAGCGCGGCTATGTGTTCGTCGGCTACCACGGCACCTTCCTCGAAGCGGCGCAAAGCATC
GluArgGlyTyrValPheValGlyTyrHisGlyThrPheLeuGluAlaAlaGlnSerIle

910 930 950
GTCTTCGGCGGGGTGCGCGCGCAGCCAGGACCTCGACGCGATCTGGCGCGGTTTCTAT
ValPheGlyGlyValArgAlaArgSerGlnAspLeuAspAlaIleTrpArgGlyPheTyr
970 990 1010
ATCGCCGGCGATCCGGCGCTGGCCTACGGCTACGCCCAGGACCAGGAACCCGACGCACGC
IleAlaGlyAspProAlaLeuAlaTyrGlyTyrAlaGlnAspGlnGluProAspAlaArg
1030 1050 1070
GGCCGGATCCGCAACGGTGCCCTGCTGCGGGTCTATGTGCCGCGCTCGAGCCTGCCGGGC
GlyArgIleArgAsnGlyAlaLeuLeuArgValTyrValProArgSerSerLeuProGly
1090 1110 1130
TTCTACCGCACCAGCCTGACCCTGGCCGCGCCGGAGGCGGCGGGCGAGGTCGAACGGCTG
PheTyrArgThrSerLeuThrLeuAlaAlaProGluAlaAlaGlyGluValGluArgLeu
1150 1170 1190
ATCGGCCATCCGCTGCCGCTGCGCCTGGACGCCATCACCGGCCCCGAGGAGGAAGGCGGG
IleGlyHisProLeuProLeuArgLeuAspAlaIleThrGlyProGluGluGluGlyGly
1210 1230 1250
CGCCTGGAGACCATTCTCGGCTGGCCGCTGGCCGAGCGCACCGTGGTGATTCCCTCGGCG
ArgLeuGluThrIleLeuGlyTrpProLeuAlaGluArgThrValValIleProSerAla
1270 1290 1310
ATCCCCACCGACCCGCGCAACGTCGGCGGCGACCTCGACCCGTCCAGCATCCCCGACAAG
IleProThrAspProArgAsnValGlyGlyAspLeuAspProSerSerIleProAspLys
1330 1350 1370
GAACAGGCGATCAGCGCCCTGCCGGACTACGCCAGCCAGCCCGGCAAACCGCCGCGCGAG
GluGlnAlaIleSerAlaLeuProAspTyrAlaSerGlnProGlyLysProProArgGlu
1390 1410 1430
GACCTGAAGTAACTGCCGCGACCGGCCGGCTCCCTTCGCAGGAGCCGGCCTTCTCGGGGC
AspLeuLysEnd
1450 1470 1490
CTGGCCATACATCAGGTTTTCCTGATGCCAGCCCAATCGAATATGAATTTCGACAGATCT

CTCGAG

SEQ ID NO:. 11 and 12
SEQUENCE TYPE: nucleotide
SEQUENCE LENGTH: 2214 bp
MOLECULE TYPE: plasmid DNA
ORIGINAL SOURCE ORGANISM: human; mouse; P. aeruginosa; synthetic
IMMEDIATE EXPERIMENTAL SOURCE: E.coli
NAME OF CELL CLONE: pMS238-5-TGF

FEATURES: from 1 to 63 bp ompA signal peptide
from 64 to 114 bp synthetic spacer
from 115 to 834 bp scFv(FRP5) cDNA
from 835 to 867 bp synthetic spacer
from 868 to 1212 bp ETA gene amino acids 252 to 366
from 1213 to 1230 bp synthetic spacer
from 1230 to 1380 bp human TGF-α cDNA
from 1381 to 1395 bp synthetic spacer
from 1396 to 2100 bp ETA gene amino acids 380 to 613

PROPERTIES: encodes scFv(FRP5)/TGFα-ETA

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGCAGTCTGGACCTGAACTGAAGAAGCCTGGAGAGACAGTCAAGATCTCCTGC
GlnLeuGlnGlnSerGlyProGluLeuLysLysProGlyGluThrValLysIleSerCys
190 210 230
AAGGCCTCTGGGTATCCTTTCACAAACTATGGAATGAACTGGGTGAAGCAGGCTCCAGGA
LysAlaSerGlyTyrProPheThrAsnTyrGlyMetAsnTrpValLysGlnAlaProGly
250 270 290
CAGGGTTTAAAGTGGATGGGCTGGATTAACACCTCCACTGGAGAGTCAACATTTGCTGAT
GlnGlyLeuLysTrpMetGlyTrpIleAsnThrSerThrGlyGluSerThrPheAlaAsp
310 330 350
GACTTCAAGGGACGGTTTGACTTCTCTTTGGAAACCTCTGCCAACACTGCCTATTTGCAG
AspPheLysGlyArgPheAspPheSerLeuGluThrSerAlaAsnThrAlaTyrLeuGln
370 390 410
ATCAACAACCTCAAAAGTGAAGACATGGCTACATATTTCTGTGCAAGATGGGAGGTTTAC
IleAsnAsnLeuLysSerGluAspMetAlaThrTyrPheCysAlaArgTrpGluValTyr
430 450 470
CACGGCTACGTTCCTTACTGGGGCCAAGGGACCACGGTCACCGTTTCCTCTGGCGGTGGC
HisGlyTyrValProTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCAC
GlySerGlyGlyGlyGlySerGlyGlyGlyGlySerAspIleGlnLeuThrGlnSerHis
550 570 590
AAATTCCTGTCCACTTCAGTAGGAGACAGGGTCAGCATCACCTGCAAGGCCAGTCAGGAT
LysPheLeuSerThrSerValGlyAspArgValSerIleThrCysLysAlaSerGlnAsp
610 630 650
GTGTATAATGCTGTTGCCTGGTATCAACAGAAACCAGGACAATCTCCTAAACTTCTGATT
ValTyrAsnAlaValAlaTrpTyrGlnGlnLysProGlyGlnSerProLysLeuLeuIle
670 690 710
TACTCGGCATCCTCCCGGTACACTGGAGTCCCTTCTCGCTTCACTGGCAGTGGCTCTGGG
TyrSerAlaSerSerArgTyrThrGlyValProSerArgPheThrGlySerGlySerGly
730 750 770
CCGGATTTCACTTTCACCATCAGCAGTGTGCAGGCTGAAGACCTGGCAGTTTATTTCTGT
ProAspPheThrPheThrIleSerSerValGlnAlaGluAspLeuAlaValTyrPheCys
790 810 830
CAGCAACATTTTCGTACTCCATTCACGTTCGGCTCGGGGACAAAATTGGAGATCAAAGCT
GlnGlnHisPheArgThrProPheThrPheGlySerGlyThrLysLeuGluIleLysAla

850 870 890
CTAGAGCACCATCATCACCATCACCTAGAGGGCGGCAGCCTGGCCGCGCTGACCGCGCAC
LeuGluHisHisHisHisHisHisLeuGluGlyGlySerLeuAlaAlaLeuThrAlaHis
910 930 950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970 990 1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030 1050 1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090 1110 1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150 1170 1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210 1230 1250
GCCAACGCCGACGAGAAGCTTGGTACCGGTGTGGTGTCCCATTTTAATGACTGCCCAGAT
AlaAsnAlaAspGluLysLeuGlyThrGlyValValSerHisPheAsnAspCysProAsp
1270 1290 1310
TCCCACACTCAGTTCTGCTTCCATGGAACCTGCAGGTTTTTGGTGCAGGAGGACAAGCCA
SerHisThrGlnPheCysPheHisGlyThrCysArgPheLeuValGlnGluAspLysPro
1330 1350 1370
GCATGTGTCTGCCATTCTGGGTACGTTGGTGCACGCTGTGAGCATGCGGACCTCCTGGCC
AlaCysValCysHisSerGlyTyrValGlyAlaArgCysGluHisAlaAspLeuLeuAla
1390 1410 1430
TCTCTAGAGAGCTCTGCGGGCCCGGCGGACAGCGGCGACGCCCTGCTGGAGCGCAACTAT
SerLeuGluSerSerAlaGlyProAlaAspSerGlyAspAlaLeuLeuGluArgAsnTyr
1450 1470 1490
CCCACTGGCGCGGAGTTCCTCGGCGACGGCGGCGACGTCAGCTTCAGCACCCGCGGCACG
ProThrGlyAlaGluPheLeuGlyAspGlyGlyAspValSerPheSerThrArgGlyThr
1510 1530 1550
CAGAACTGGACGGTGGAGCGGCTGCTCCAGGCGCACCGCCAACTGGAGGAGCGCGGCTAT
GlnAsnTrpThrValGluArgLeuLeuGlnAlaHisArgGlnLeuGluGluArgGlyTyr
1570 1590 1610
GTGTTCGTCGGCTACCACGGCACCTTCCTCGAAGCGGCGCAAAGCATCGTCTTCGGCGGG
ValPheValGlyTyrHisGlyThrPheLeuGluAlaAlaGlnSerIleValPheGlyGly
1630 1650 1670
GTGCGCGCGCGCAGCCAGGACCTCGACGCGATCTGGCGCGGTTTCTATATCGCCGGCGAT
ValArgAlaArgSerGlnAspLeuAspAlaIleTrpArgGlyPheTyrIleAlaGlyAsp
1690 1710 1730
CCGGCGCTGGCCTACGGCTACGCCCAGGACCAGGAACCCGACGCACGCGGCCGGATCCGC
ProAlaLeuAlaTyrGlyTyrAlaGlnAspGlnGluProAspAlaArgGlyArgIleArg
1750 1770 1790
AACGGTGCCCTGCTGCGGGTCTATGTGCCGCGCTCGAGCCTGCCGGGCTTCTACCGCACC
AsnGlyAlaLeuLeuArgValTyrValProArgSerSerLeuProGlyPheTyrArgThr
1810 1830 1850
AGCCTGACCCTGGCCGCGCCGGAGGCGGCGGGCGAGGTCGAACGGCTGATCGGCCATCCG
SerLeuThrLeuAlaAlaProGluAlaAlaGlyGluValGluArgLeuIleGlyHisPro
1870 1890 1910
CTGCCGCTGCGCCTGGACGCCATCACCGGCCCCGAGGAGGAAGGCGGGCGCCTGGAGACC
LeuProLeuArgLeuAspAlaIleThrGlyProGluGluGluGlyGlyArgLeuGluThr
1930 1950 1970
ATTCTCGGCTGGCCGCTGGCCGAGCGCACCGTGGTGATTCCCTCGGCGATCCCCACCGAC
IleLeuGlyTrpProLeuAlaGluArgThrValValIleProSerAlaIleProThrAsp
1990 2010 2030
CCGCGCAACGTCGGCGGCGACCTCGACCCGTCCAGCATCCCCGACAAGGAACAGGCGATC
ProArgAsnValGlyGlyAspLeuAspProSerSerIleProAspLysGluGlnAlaIle
2050 2070 2090
AGCGCCCTGCCGGACTACGCCAGCCAGCCCGGCAAACCGCCGCGCGAGGACCTGAAGTAA
SerAlaLeuProAspTyrAlaSerGlnProGlyLysProProArgGluAspLeuLysEnd

2110 2130 2150
CTGCCGCGACCGGCCGGCTCCCTTCGCAGGAGCCGGCCTTCTCGGGGCCTGGCCATACAT
2170 2190 2210
CAGGTTTTCCTGATGCCAGCCCAATCGAATATGAATTTCGACAGATCTCTCGAG

SEQ ID NO: 13 and 14
SEQUENCE TYPE: nucleotide
SEQUENCE LENGTH: 2793 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pMS238-5-225

FEATURES: from 1 to 63 bp ompA signal peptide
from 64 to 114 bp synthetic spacer
from 115 to 834 bp scFv(FRP5) cDNA
from 835 to 867 bp synthetic spacer
from 868 to 1212 bp ETA gene amino acids 252 to 366
from 1213 to 1221 bp synthetic spacer
from 1222 to 1941 bp scFv(225) cDNA
from 1942 to 1974 bp synthetic spacer
from 1975 to 2679 bp ETA gene amino acids 380 to 613

PROPERTIES: encodes scFv2(FRP5/225)-ETA (version 1)

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGCAGTCTGGACCTGAACTGAAGAAGCCTGGAGAGACAGTCAAGATCTCCTGC
GlnLeuGlnGlnSerGlyProGluLeuLysLysProGlyGluThrValLysIleSerCys
190 210 230
AAGGCCTCTGGGTATCCTTTCACAAACTATGGAATGAACTGGGTGAAGCAGGCTCCAGGA
LysAlaSerGlyTyrProPheThrAsnTyrGlyMetAsnTrpValLysGlnAlaProGly
250 270 290
CAGGGTTTAAAGTGGATGGGCTGGATTAACACCTCCACTGGAGAGTCAACATTTGCTGAT
GlnGlyLeuLysTrpMetGlyTrpIleAsnThrSerThrGlyGluSerThrPheAlaAsp
310 330 350
GACTTCAAGGGACGGTTTGACTTCTCTTTGGAAACCTCTGCCAACACTGCCTATTTGCAG
AspPheLysGlyArgPheAspPheSerLeuGluThrSerAlaAsnThrAlaTyrLeuGln
370 390 410
ATCAACAACCTCAAAAGTGAAGACATGGCTACATATTTCTGTGCAAGATGGGAGGTTTAC
IleAsnAsnLeuLysSerGluAspMetAlaThrTyrPheCysAlaArgTrpGluValTyr
430 450 470
CACGGCTACGTTCCTTACTGGGGCCAAGGGACCACGGTCACCGTTTCCTCTGGCGGTGGC
HisGlyTyrValProTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCAC
GlySerGlyGlyGlyGlySerGlyGlyGlyGlySerAspIleGlnLeuThrGlnSerHis
550 570 590
AAATTCCTGTCCACTTCAGTAGGAGACAGGGTCAGCATCACCTGCAAGGCCAGTCAGGAT
LysPheLeuSerThrSerValGlyAspArgValSerIleThrCysLysAlaSerGlnAsp
610 630 650
GTGTATAATGCTGTTGCCTGGTATCAACAGAAACCAGGACAATCTCCTAAACTTCTGATT
ValTyrAsnAlaValAlaTrpTyrGlnGlnLysProGlyGlnSerProLysLeuLeuIle
670 690 710
TACTCGGCATCCTCCCGGTACACTGGAGTCCCTTCTCGCTTCACTGGCAGTGGCTCTGGG
TyrSerAlaSerSerArgTyrThrGlyValProSerArgPheThrGlySerGlySerGly
730 750 770
CCGGATTTCACTTTCACCATCAGCAGTGTGCAGGCTGAAGACCTGGCAGTTTATTTCTGT
ProAspPheThrPheThrIleSerSerValGlnAlaGluAspLeuAlaValTyrPheCys
790 810 830
CAGCAACATTTTCGTACTCCATTCACGTTCGGCTCGGGGACAAAATTGGAGATCAAAGCT
GlnGlnHisPheArgThrProPheThrPheGlySerGlyThrLysLeuGluIleLysAla

850 870 890
CTAGAGCACCATCATCACCATCACCTAGAGGGCGGCAGCCTGGCCGCGCTGACCGCGCAC
LeuGluHisHisHisHisHisHisLeuGluGlyGlySerLeuAlaAlaLeuThrAlaHis
910 930 950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970 990 1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030 1050 1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090 1110 1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150 1170 1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210 1230 1250
GCCAACGCCGACGAGAAGCTTCAGGTACAACTGCAGGAGTCAGGACCTGGCCTAGTGCAG
AlaAsnAlaAspGluLysLeuGlnValGlnLeuGlnGluSerGlyProGlyLeuValGln
1270 1290 1310
CCCTCACAGAGCCTGTCCATCACCTGCACAGTCTCTGGTTTCTCATTAACTAACTATGGT
ProSerGlnSerLeuSerIleThrCysThrValSerGlyPheSerLeuThrAsnTyrGly
1330 1350 1370
GTACACTGGGTTCGCCAGTCTCCAGGAAAGGGTCTGGAGTGGCTGGGAGTGATATGGAGT
ValHisTrpValArgGlnSerProGlyLysGlyLeuGluTrpLeuGlyValIleTrpSer
1390 1410 1430
GGTGGAAACACAGACTATAATACACCTTTCACATCCAGACTGAGCATCAACAAGGACAAT
GlyGlyAsnThrAspTyrAsnThrProPheThrSerArgLeuSerIleAsnLysAspAsn
1450 1470 1490
TCCAAGAGCCAAGTTTTCTTTAAAATGAACAGTCTGCAATCTAATGACACAGCCATATAT
SerLysSerGlnValPhePheLysMetAsnSerLeuGlnSerAsnAspThrAlaIleTyr
1510 1530 1550
TACTGTGCCAGAGCCCTCACCTACTATGATTACGAGTTTGCTTACTGGGGCCAAGGGACC
TyrCysAlaArgAlaLeuThrTyrTyrAspTyrGluPheAlaTyrTrpGlyGlnGlyThr
1570 1590 1610
ACGGTCACCGTTTCCTCTGGCGGTGGCGGTTCTGGTGGCGGTGGCTCCGGCGGTGGCGGT
ThrValThrValSerSerGlyGlyGlyGlySerGlyGlyGlyGlySerGlyGlyGlyGly
1630 1650 1670
TCTGACATCCAGCTGACCCAGTCTCCAGTCATCCTGTCTGTGAGTCCAGGAGAAAGAGTC
SerAspIleGlnLeuThrGlnSerProValIleLeuSerValSerProGlyGluArgVal
1690 1710 1730
AGTTTCTCCTGCAGGGCCAGTCAGAGTATTGGCACAAACATACACTGGTATCAGCAAAGA
SerPheSerCysArgAlaSerGlnSerIleGlyThrAsnIleHisTrpTyrGlnGlnArg
1750 1770 1790
ACAAATGGTTCTCCAAGGCTTCTCATAAAGTATGCTTCTGAGTCTATCTCTGGGATCCCT
ThrAsnGlySerProArgLeuLeuIleLysTyrAlaSerGluSerIleSerGlyIlePro
1810 1830 1850
TCCAGGTTTAGTGGCAGTGGATCAGGGACAGATTTTACTCTTAGCATCAACAGTGTGGAG
SerArgPheSerGlySerGlySerGlyThrAspPheThrLeuSerIleAsnSerValGlu
1870 1890 1910
TCTGAAGATATTGCAGATTATTATTGTCAACAAAATAATAACTGGCCAACCACGTTCGGT
SerGluAspIleAlaAspTyrTyrCysGlnGlnAsnAsnAsnTrpProThrThrPheGly
1930 1950 1970
GCTGGGACCAAGCTGGAGATCAAAGCTCTAGAGGATCCCCGGGTACCGAGCTCTGCGGGC
AlaGlyThrLysLeuGluIleLysAlaLeuGluAspProArgValProSerSerAlaGly
1990 2010 2030
CCGGCGGACAGCGGCGACGCCCTGCTGGAGCGCAACTATCCCACTGGCGCGGAGTTCCTC
ProAlaAspSerGlyAspAlaLeuLeuGluArgAsnTyrProThrGlyAlaGluPheLeu
2050 2070 2090
GGCGACGGCGGCGACGTCAGCTTCAGCACCCGCGGCACGCAGAACTGGACGGTGGAGCGG
GlyAspGlyGlyAspValSerPheSerThrArgGlyThrGlnAsnTrpThrValGluArg

2110 2130 2150
CTGCTCCAGGCGCACCGCCAACTGGAGGAGCGCGGCTATGTGTTCGTCGGCTACCACGGC
LeuLeuGlnAlaHisArgGlnLeuGluGluArgGlyTyrValPheValGlyTyrHisGly
2170 2190 2210
ACCTTCCTCGAAGCGGCGCAAAGCATCGTCTTCGGCGGGGTGCGCGCGCAGCCAGGAC
ThrPheLeuGluAlaAlaGlnSerIleValPheGlyGlyValArgAlaArgSerGlnAsp
2230 2250 2270
CTCGACGCGATCTGGCGCGGTTTCTATATCGCCGGCGATCCGGCGCTGGCCTACGGCTAC
LeuAspAlaIleTrpArgGlyPheTyrIleAlaGlyAspProAlaLeuAlaTyrGlyTyr
2290 2310 2330
GCCCAGGACCAGGAACCCGACGCACGCGGCCGGATCCGCAACGGTGCCCTGCTGCGGGTC
AlaGlnAspGlnGluProAspAlaArgGlyArgIleArgAsnGlyAlaLeuLeuArgVal
2350 2370 2390
TATGTGCCGCGCTCGAGCCTGCCGGGCTTCTACCGCACCAGCCTGACCCTGGCCGCGCCG
TyrValProArgSerSerLeuProGlyPheTyrArgThrSerLeuThrLeuAlaAlaPro
2410 2430 2450
GAGGCGGCGGGCGAGGTCGAACGGCTGATCGGCCATCCGCTGCCGCTGCGCCTGGACGCC
GluAlaAlaGlyGluValGluArgLeuIleGlyHisProLeuProLeuArgLeuAspAla
2470 2490 2510
ATCACCGGCCCCGAGGAGGAAGGCGGGCGCCTGGAGACCATTCTCGGCTGGCCGCTGGCC
IleThrGlyProGluGluGluGlyGlyArgLeuGluThrIleLeuGlyTrpProLeuAla
2530 2550 2570
GAGCGCACCGTGGTGATTCCCTCGGCGATCCCCACCGACCCGCGCAACGTCGGCGGCGAC
GluArgThrValValIleProSerAlaIleProThrAspProArgAsnValGlyGlyAsp
2590 2610 2630
CTCGACCCGTCCAGCATCCCCGACAAGGAACAGGCGATCAGCGCCCTGCCGGACTACGCC
LeuAspProSerSerIleProAspLysGluGlnAlaIleSerAlaLeuProAspTyrAla
2650 2670 2690
AGCCAGCCCGGCAAACCGCCGCGCGAGGACCTGAAGTAACTGCCGCGACCGGCCGGCTCC
SerGlnProGlyLysProProArgGluAspLeuLysEnd
2710 2730 2750
CTTCGCAGGAGCCGGCCTTCTCGGGGCCTGGCCATACATCAGGTTTTCCTGATGCCAGCC
2770 2790
CAATCGAATATGAATTTCGACAGATCTCTCGAG

SEQ ID NO: 15 and 16
SEQUENCE TYPE: nucleotide
SEQUENCE LENGTH: 2793 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pMS238-225-5

FEATURES: from 1 to 63 bp ompA signal peptide
from 64 to 114 bp synthetic spacer
from 115 to 834 bp scFv(225) cDNA
from 835 to 867 bp synthetic spacer
from 868 to 1212 bp ETA gene amino acids 252 to 366
from 1213 to 1221 bp synthetic spacer
from 1222 to 1941 bp scFv(FRP5) cDNA
from 1942 to 1974 bp synthetic spacer
from 1975 to 2679 bp ETA gene amino acids 380 to 613

PROPERTIES: encodes scFv2(225/FRP5)-ETA

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGGAGTCAGGACCTGGCCTAGTGCAGCCCTCACAGAGCCTGTCCATCACCTGC
GlnLeuGlnGluSerGlyProGlyLeuValGlnProSerGlnSerLeuSerIleThrCys
190 210 230
ACAGTCTCTGGTTTCTCATTAACTAACTATGGTGTACACTGGGTTCGCCAGTCTCCAGGA
ThrValSerGlyPheSerLeuThrAsnTyrGlyValHisTrpValArgGlnSerProGly
250 270 290
AAGGGTCTGGAGTGGCTGGGAGTGATATGGAGTGGTGGAAACACAGACTATAATACACCT
LysGlyLeuGluTrpLeuGlyValIleTrpSerGlyGlyAsnThrAspTyrAsnThrPro
310 330 350
TTCACATCCAGACTGAGCATCAACAAGGACAATTCCAAGAGCCAAGTTTTCTTTAAAATG
PheThrSerArgLeuSerIleAsnLysAspAsnSerLysSerGlnValPhePheLysMet
370 390 410
AACAGTCTGCAATCTAATGACACAGCCATATATTACTGTGCCAGAGCCCTCACCTACTAT
AsnSerLeuGlnSerAsnAspThrAlaIleTyrTyrCysAlaArgAlaLeuThrTyrTyr
430 450 470
GATTACGAGTTTGCTTACTGGGGCCAAGGGACCACGGTCACCGTTTCCTCTGGCGGTGGC
AspTyrGluPheAlaTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCCA
GlySerGlyGlyGlyGlySerGlyGlyGlyGlySerAspIleGlnLeuThrGlnSerPro
550 570 590
GTCATCCTGTCTGTGAGTCCAGGAGAAAGAGTCAGTTTCTCCTGCAGGGCCAGTCAGAGT
ValIleLeuSerValSerProGlyGluArgValSerPheSerCysArgAlaSerGlnSer
610 630 650
ATTGGCACAAACATACACTGGTATCAGCAAAGAACAAATGGTTCTCCAAGGCTTCTCATA
IleGlyThrAsnIleHisTrpTyrGlnGlnArgThrAsnGlySerProArgLeuLeuIle
670 690 710
AAGTATGCTTCTGAGTCTATCTCTGGGATCCCTTCCAGGTTTAGTGGCAGTGGATCAGGG
LysTyrAlaSerGluSerIleSerGlyIleProSerArgPheSerGlySerGlySerGly
730 750 770
ACAGATTTTACTCTTAGCATCAACAGTGTGGAGTCTGAAGATATTGCAGATTATTATTGT
ThrAspPheThrLeuSerIleAsnSerValGluSerGluAspIleAlaAspTyrTyrCys
790 810 830
CAACAAAATAATAACTGGCCAACCACGTTCGGTGCTGGGACCAAGCTGGAGATCAAAGCT
GlnGlnAsnAsnAsnTrpProThrThrPheGlyAlaGlyThrLysLeuGluIleLysAla

850                    870                    890
CTAGAGCACCATCATCACCATCACCTAGAGGGCGGCAGCCTGGCCGCGCTGACCGCGCAC
LeuGluHisHisHisHisHisHisLeuGluGlyGlySerLeuAlaAlaLeuThrAlaHis
910                    930                    950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970                    990                    1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030                   1050                   1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090                   1110                   1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150                   1170                   1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210                   1230                   1250
GCCAACGCCGACGAGAAGCTTCAGGTACAACTGCAGCAGTCTGGACCTGAACTGAAGAAG
AlaAsnAlaAspGluLysLeuGlnValGlnLeuGlnGlnSerGlyProGluLeuLysLys
1270                   1290                   1310
CCTGGAGAGACAGTCAAGATCTCCTGCAAGGCCTCTGGGTATCCTTTCACAAACTATGGA
ProGlyGluThrValLysIleSerCysLysAlaSerGlyTyrProPheThrAsnTyrGly
1330                   1350                   1370
ATGAACTGGGTGAAGCAGGCTCCAGGACAGGGTTTAAAGTGGATGGGCTGGATTAACACC
MetAsnTrpValLysGlnAlaProGlyGlnGlyLeuLysTrpMetGlyTrpIleAsnThr
1390                   1410                   1430
TCCACTGGAGAGTCAACATTTGCTGATGACTTCAAGGGACGGTTTGACTTCTCTTTGGAA
SerThrGlyGluSerThrPheAlaAspAspPheLysGlyArgPheAspPheSerLeuGlu
1450                   1470                   1490
ACCTCTGCCAACACTGCCTATTTGCAGATCAACAACCTCAAAAGTGAAGACATGGCTACA
ThrSerAlaAsnThrAlaTyrLeuGlnIleAsnAsnLeuLysSerGluAspMetAlaThr
1510                   1530                   1550
TATTTCTGTGCAAGATGGGAGGTTTACCACGGCTACGTTCCTTACTGGGGCCAAGGGACC
TyrPheCysAlaArgTrpGluValTyrHisGlyTyrValProTyrTrpGlyGlnGlyThr
1570                   1590                   1610
ACGGTCACCGTTTCCTCTGGCGGTGGCGGTTCTGGTGGCGGTGGCTCCGGCGGTGGCGGT
ThrValThrValSerSerGlyGlyGlyGlySerGlyGlyGlyGlySerGlyGlyGlyGly
1630                   1650                   1670
TCTGACATCCAGCTGACCCAGTCTCACAAATTCCTGTCCACTTCAGTAGGAGACAGGGTC
SerAspIleGlnLeuThrGlnSerHisLysPheLeuSerThrSerValGlyAspArgVal
1690                   1710                   1730
AGCATCACCTGCAAGGCCAGTCAGGATGTGTATAATGCTGTTGCCTGGTATCAACAGAAA
SerIleThrCysLysAlaSerGlnAspValTyrAsnAlaValAlaTrpTyrGlnGlnLys
1750                   1770                   1790
CCAGGACAATCTCCTAAACTTCTGATTTACTCGGCATCCTCCCGGTACACTGGAGTCCCT
ProGlyGlnSerProLysLeuLeuIleTyrSerAlaSerSerArgTyrThrGlyValPro
1810                   1830                   1850
TCTCGCTTCACTGGCAGTGGCTCTGGGCCGGATTTCACTTTCACCATCAGCAGTGTGCAG
SerArgPheThrGlySerGlySerGlyProAspPheThrPheThrIleSerSerValGln
1870                   1890                   1910
GCTGAAGACCTGGCAGTTTATTTCTGTCAGCAACATTTTCGTACTCCATTCACGTTCGGC
AlaGluAspLeuAlaValTyrPheCysGlnGlnHisPheArgThrProPheThrPheGly
1930                   1950                   1970
TCGGGGACAAAATTGGAGATCAAAGCTCTAGAGGATCCCCGGGTACCGAGCTCTGCGGGC
SerGlyThrLysLeuGluIleLysAlaLeuGluAspProArgValProSerSerAlaGly
1990                   2010                   2030
CCGGCGGACAGCGGCGACGCCCTGCTGGAGCGCAACTATCCCACTGGCGCGGAGTTCCTC
ProAlaAspSerGlyAspAlaLeuLeuGluArgAsnTyrProThrGlyAlaGluPheLeu
2050                   2070                   2090
GGCGACGGCGGCGACGTCAGCTTCAGCACCCGCGGCACGCAGAACTGGACGGTGGAGCGG
GlyAspGlyGlyAspValSerPheSerThrArgGlyThrGlnAsnTrpThrValGluArg

2110                    2130                    2150
CTGCTCCAGGCGCACCGCCAACTGGAGGAGCGCGGCTATGTGTTCGTCGGCTACCACGGC
LeuLeuGlnAlaHisArgGlnLeuGluGluArgGlyTyrValPheValGlyTyrHisGly
        2170                    2190                    2210
ACCTTCCTCGAAGCGGCGCAAAGCATCGTCTTCGGCGGGGTGCGCGCGCAGCCAGGAC
ThrPheLeuGluAlaAlaGlnSerIleValPheGlyGlyValArgAlaArgSerGlnAsp
        2230                    2250                    2270
CTCGACGCGATCTGGCGCGGTTTCTATATCGCCGGCGATCCGGCGCTGGCCTACGGCTAC
LeuAspAlaIleTrpArgGlyPheTyrIleAlaGlyAspProAlaLeuAlaTyrGlyTyr
        2290                    2310                    2330
GCCCAGGACCAGGAACCCGACGCACGCGGCCGGATCCGCAACGGTGCCCTGCTGCGGGTC
AlaGlnAspGlnGluProAspAlaArgGlyArgIleArgAsnGlyAlaLeuLeuArgVal
        2350                    2370                    2390
TATGTGCCGCGCTCGAGCCTGCCGGGCTTCTACCGCACCAGCCTGACCCTGGCCGCGCCG
TyrValProArgSerSerLeuProGlyPheTyrArgThrSerLeuThrLeuAlaAlaPro
        2410                    2430                    2450
GAGGCGGCGGGCGAGGTCGAACGGCTGATCGGCCATCCGCTGCCGCTGCGCCTGGACGCC
GluAlaAlaGlyGluValGluArgLeuIleGlyHisProLeuProLeuArgLeuAspAla
        2470                    2490                    2510
ATCACCGGCCCCGAGGAGGAAGGCGGGCGCCTGGAGACCATTCTCGGCTGGCCGCTGGCC
IleThrGlyProGluGluGluGlyGlyArgLeuGluThrIleLeuGlyTrpProLeuAla
        2530                    2550                    2570
GAGCGCACCGTGGTGATTCCCTCGGCGATCCCCACCGACCCGCGCAACGTCGGCGGCGAC
GluArgThrValValIleProSerAlaIleProThrAspProArgAsnValGlyGlyAsp
        2590                    2610                    2630
CTCGACCCGTCCAGCATCCCCGACAAGGAACAGGCGATCAGCGCCCTGCCGGACTACGCC
LeuAspProSerSerIleProAspLysGluGlnAlaIleSerAlaLeuProAspTyrAla
        2650                    2670                    2690
AGCCAGCCCGGCAAACCGCCGCGCGAGGACCTGAAGTAACTGCCGCGACCGGCCGGCTCC
SerGlnProGlyLysProProArgGluAspLeuLysEnd
        2710                    2730                    2750
CTTCGCAGGAGCCGGCCTTCTCGGGGCCTGGCCATACATCAGGTTTTCCTGATGCCAGCC
        2770                    2790
CAATCGAATATGAATTTCGACAGATCTCTCGAG

SEQ ID NO: 17 and 18
SEQUENCE TYPE: nucleotide
SEQUENCE LENGTH: 3177 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pMS240-5-225

FEATURES: from 1 to 63 bp ompA signal peptide
from 64 to 114 bp synthetic spacer
from 115 to 834 bp scFv(FRP5) cDNA
from 835 to 867 bp synthetic spacer
from 868 to 1212 bp ETA gene amino acids 252 to 366
from 1213 to 1221 bp synthetic spacer
from 1222 to 1941 bp scFv(225) cDNA
from 1942 to 1974 bp synthetic spacer
from 1975 to 3063 bp ETA gene amino acids 252 to 613

PROPERTIES: encodes scFv2(FRP5/225)-ETA (version 2; so far best version)

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGCAGTCTGGACCTGAACTGAAGAAGCCTGGAGAGACAGTCAAGATCTCCTGC
GlnLeuGlnGlnSerGlyProGluLeuLysLysProGlyGluThrValLysIleSerCys
190 210 230
AAGGCCTCTGGGTATCCTTTCACAAACTATGGAATGAACTGGGTGAAGCAGGCTCCAGGA
LysAlaSerGlyTyrProPheThrAsnTyrGlyMetAsnTrpValLysGlnAlaProGly
250 270 290
CAGGGTTTAAAGTGGATGGGCTGGATTAACACCTCCACTGGAGAGTCAACATTTGCTGAT
GlnGlyLeuLysTrpMetGlyTrpIleAsnThrSerThrGlyGluSerThrPheAlaAsp
310 330 350
GACTTCAAGGGACGGTTTGACTTCTCTTTGGAAACCTCTGCCAACACTGCCTATTTGCAG
AspPheLysGlyArgPheAspPheSerLeuGluThrSerAlaAsnThrAlaTyrLeuGln
370 390 410
ATCAACAACCTCAAAAGTGAAGACATGGCTACATATTTCTGTGCAAGATGGGAGGTTTAC
IleAsnAsnLeuLysSerGluAspMetAlaThrTyrPheCysAlaArgTrpGluValTyr
430 450 470
CACGGCTACGTTCCTTACTGGGGCCAAGGGACCACGGTCACCGTTTCCTCTGGCGGTGGC
HisGlyTyrValProTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCAC
GlySerGlyGlyGlyGlySerGlyGlyGlyGlySerAspIleGlnLeuThrGlnSerHis
550 570 590
AAATTCCTGTCCACTTCAGTAGGAGACAGGGTCAGCATCACCTGCAAGGCCAGTCAGGAT
LysPheLeuSerThrSerValGlyAspArgValSerIleThrCysLysAlaSerGlnAsp
610 630 650
GTGTATAATGCTGTTGCCTGGTATCAACAGAAACCAGGACAATCTCCTAAACTTCTGATT
ValTyrAsnAlaValAlaTrpTyrGlnGlnLysProGlyGlnSerProLysLeuLeuIle
670 690 710
TACTCGGCATCCTCCCGGTACACTGGAGTCCCTTCTCGCTTCACTGGCAGTGGCTCTGGG
TyrSerAlaSerSerArgTyrThrGlyValProSerArgPheThrGlySerGlySerGly
730 750 770
CCGGATTTCACTTTCACCATCAGCAGTGTGCAGGCTGAAGACCTGGCAGTTTATTTCTGT
ProAspPheThrPheThrIleSerSerValGlnAlaGluAspLeuAlaValTyrPheCys
790 810 830
CAGCAACATTTTCGTACTCCATTCACGTTCGGCTCGGGGACAAAATTGGAGATCAAAGCT
GlnGlnHisPheArgThrProPheThrPheGlySerGlyThrLysLeuGluIleLysAla

850 870 890
CTAGAGCACCATCATCACCATCACCTAGAGGGCGGCAGCCTGGCCGCGCTGACCGCGCAC
LeuGluHisHisHisHisHisHisLeuGluGlyGlySerLeuAlaAlaLeuThrAlaHis
910 930 950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970 990 1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030 1050 1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090 1110 1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150 1170 1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210 1230 1250
GCCAACGCCGACGAGAAGCTTCAGGTACAACTGCAGGAGTCAGGACCTGGCCTAGTGCAG
AlaAsnAlaAspGluLysLeuGlnValGlnLeuGlnGluSerGlyProGlyLeuValGln
1270 1290 1310
CCCTCACAGAGCCTGTCCATCACCTGCACAGTCTCTGGTTTCTCATTAACTAACTATGGT
ProSerGlnSerLeuSerIleThrCysThrValSerGlyPheSerLeuThrAsnTyrGly
1330 1350 1370
GTACACTGGGTTCGCCAGTCTCCAGGAAAGGGTCTGGAGTGGCTGGGAGTGATATGGAGT
ValHisTrpValArgGlnSerProGlyLysGlyLeuGluTrpLeuGlyValIleTrpSer
1390 1410 1430
GGTGGAAACACAGACTATAATACACCTTTCACATCCAGACTGAGCATCAACAAGGACAAT
GlyGlyAsnThrAspTyrAsnThrProPheThrSerArgLeuSerIleAsnLysAspAsn
1450 1470 1490
TCCAAGAGCCAAGTTTTCTTTAAAATGAACAGTCTGCAATCTAATGACACAGCCATATAT
SerLysSerGlnValPhePheLysMetAsnSerLeuGlnSerAsnAspThrAlaIleTyr
1510 1530 1550
TACTGTGCCAGAGCCCTCACCTACTATGATTACGAGTTTGCTTACTGGGGCCAAGGGACC
TyrCysAlaArgAlaLeuThrTyrTyrAspTyrGluPheAlaTyrTrpGlyGlnGlyThr
1570 1590 1610
ACGGTCACCGTTTCCTCTGGCGGTGGCGGTTCTGGTGGCGGTGGCTCCGGCGGTGGCGGT
ThrValThrValSerSerGlyGlyGlyGlySerGlyGlyGlyGlySerGlyGlyGlyGly
1630 1650 1670
TCTGACATCCAGCTGACCCAGTCTCCAGTCATCCTGTCTGTGAGTCCAGGAGAAAGAGTC
SerAspIleGlnLeuThrGlnSerProValIleLeuSerValSerProGlyGluArgVal
1690 1710 1730
AGTTTCTCCTGCAGGGCCAGTCAGAGTATTGGCACAAACATACACTGGTATCAGCAAAGA
SerPheSerCysArgAlaSerGlnSerIleGlyThrAsnIleHisTrpTyrGlnGlnArg
1750 1770 1790
ACAAATGGTTCTCCAAGGCTTCTCATAAAGTATGCTTCTGAGTCTATCTCTGGGATCCCT
ThrAsnGlySerProArgLeuLeuIleLysTyrAlaSerGluSerIleSerGlyIlePro
1810 1830 1850
TCCAGGTTTAGTGGCAGTGGATCAGGGACAGATTTTACTCTTAGCATCAACAGTGTGGAG
SerArgPheSerGlySerGlySerGlyThrAspPheThrLeuSerIleAsnSerValGlu
1870 1890 1910
TCTGAAGATATTGCAGATTATTATTGTCAACAAAATAATAACTGGCCAACCACGTTCGGT
SerGluAspIleAlaAspTyrTyrCysGlnGlnAsnAsnAsnTrpProThrThrPheGly
1930 1950 1970
GCTGGGACCAAGCTGGAGATCAAAGCTCTAGAGCACCATCATCACCATCACCTAGAGGGC
AlaGlyThrLysLeuGluIleLysAlaLeuGluHisHisHisHisHisHisLeuGluGly
1990 2010 2030
GGCAGCCTGGCCGCGCTGACCGCGCACCAGGCCTGCCACCTGCCGCTGGAGACTTTCACC
GlySerLeuAlaAlaLeuThrAlaHisGlnAlaCysHisLeuProLeuGluThrPheThr
2050 2070 2090
CGTCATCGCCAGCCGCGCGGCTGGGAACAACTGGAGCAGTGCGGCTATCCGGTGCAGCGG
ArgHisArgGlnProArgGlyTrpGluGlnLeuGluGlnCysGlyTyrProValGlnArg

2110 2130 2150
CTGGTCGCCCTCTACCTGGCGGCGCGACTGTCATGGAACCAGGTCGACCAGGTGATCCGC
LeuValAlaLeuTyrLeuAlaAlaArgLeuSerTrpAsnGlnValAspGlnValIleArg
2170 2190 2210
AACGCCCTGGCCAGCCCCGGCAGCGGCGGCGACCTGGGCGAAGCGATCCGCGAGCAGCCG
AsnAlaLeuAlaSerProGlySerGlyGlyAspLeuGlyGluAlaIleArgGluGlnPro
2230 2250 2270
GAGCAGGCCCGTCTGGCCCTGACCCTGGCCGCCGCCGAGAGCGAGCGCTTCGTCCGGCAG
GluGlnAlaArgLeuAlaLeuThrLeuAlaAlaAlaGluSerGluArgPheValArgGln
2290 2310 2330
GGCACCGGCAACGACGAGGCCGGCGCGGCCAACGCCGACGTGGTGAGCCTGACCTGCCCG
GlyThrGlyAsnAspGluAlaGlyAlaAlaAsnAlaAspValValSerLeuThrCysPro
2350 2370 2390
GTCGCCGCCGGTGAATGCGCGGGCCCGGCGGACAGCGGCGACGCCCTGCTGGAGCGCAAC
ValAlaAlaGlyGluCysAlaGlyProAlaAspSerGlyAspAlaLeuLeuGluArgAsn
2410 2430 2450
TATCCCACTGGCGCGGAGTTCCTCGGCGACGGCGGCGACGTCAGCTTCAGCACCCGCGGC
TyrProThrGlyAlaGluPheLeuGlyAspGlyGlyAspValSerPheSerThrArgGly
2470 2490 2510
ACGCAGAACTGGACGGTGGAGCGGCTGCTCCAGGCGCACCGCCAACTGGAGGAGCGCGGC
ThrGlnAsnTrpThrValGluArgLeuLeuGlnAlaHisArgGlnLeuGluGluArgGly
2530 2550 2570
TATGTGTTCGTCGGCTACCACGGCACCTTCCTCGAAGCGGCGCAAAGCATCGTCTTCGGC
TyrValPheValGlyTyrHisGlyThrPheLeuGluAlaAlaGlnSerIleValPheGly
2590 2610 2630
GGGGTGCGCGCGCGCAGCCAGGACCTCGACGCGATCTGGCGCGGTTTCTATATCGCCGGC
GlyValArgAlaArgSerGlnAspLeuAspAlaIleTrpArgGlyPheTyrIleAlaGly
2650 2670 2690
GATCCGGCGCTGGCCTACGGCTACGCCCAGGACCAGGAACCCGACGCACGCGGCCGGATC
AspProAlaLeuAlaTyrGlyTyrAlaGlnAspGlnGluProAspAlaArgGlyArgIle
2710 2730 2750
CGCAACGGTGCCCTGCTGCGGGTCTATGTGCCGCGCTCGAGCCTGCCGGGCTTCTACCGC
ArgAsnGlyAlaLeuLeuArgValTyrValProArgSerSerLeuProGlyPheTyrArg
2770 2790 2810
ACCAGCCTGACCCTGGCCGCGCCGGAGGCGGCGGGCGAGGTCGAACGGCTGATCGGCCAT
ThrSerLeuThrLeuAlaAlaProGluAlaAlaGlyGluValGluArgLeuIleGlyHis
2830 2850 2870
CCGCTGCCGCTGCGCCTGGACGCCATCACCGGCCCCGAGGAGGAAGGCGGGCGCCTGGAG
ProLeuProLeuArgLeuAspAlaIleThrGlyProGluGluGluGlyGlyArgLeuGlu
2890 2910 2930
ACCATTCTCGGCTGGCCGCTGGCCGAGCGCACCGTGGTGATTCCCTCGGCGATCCCCACC
ThrIleLeuGlyTrpProLeuAlaGluArgThrValValIleProSerAlaIleProThr
2950 2970 2990
GACCCGCGCAACGTCGGCGGCGACCTCGACCCGTCCAGCATCCCCGACAAGGAACAGGCG
AspProArgAsnValGlyGlyAspLeuAspProSerSerIleProAspLysGluGlnAla
3010 3030 3050
ATCAGCGCCCTGCCGGACTACGCCAGCCAGCCCGGCAAACCGCCGCGCGAGGACCTGAAG
IleSerAlaLeuProAspTyrAlaSerGlnProGlyLysProProArgGluAspLeuLys
3070 3090 3110
TAACTGCCGCGACCGGCCGGCTCCCTTCGCAGGAGCCGGCCTTCTCGGGGCCTGGCCATA
End
3130 3150 3170
CATCAGGTTTTCCTGATGCCAGCCCAATCGAATATGAATTTCGACAGATCTCTCGAG

SEQ ID NO: 19 and 20
SEQUENCE TYPE: nucleotide
SEQUENCE LENGTH: 2718 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pMS242-5-5 KDEL

FEATURES: from 1 to 63 bp ompA signal peptide
from 64 to 132 bp synthetic spacer
from 133 to 477 bp ETA gene amino acids 252 to 366
from 478 to 486 bp synthetic spacer
from 487 to 1206 bp scFv(FRP5) cDNA
from 1207 to 1239 bp synthetic spacer
from 1240 to 1932 bp ETA gene amino acids 380 to 610
from 1933 to 1937 bp synthetic spacer
from 1938 to 2658 bp scFv(FRP5) cDNA
from 2659 to 2688 bp synthetic spacer

PROPERTIES: encodes scFv2(FRP5/FRP5)-ETA (version 1)

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTTCTCGAGCAGATCTCTCTAGAGCACCATCAT
AlaAspTyrLysAspAspAspAspLysLeuLeuGluGlnIleSerLeuGluHisHisHis
130 150 170
CACCATCACCTAGAGGGCGGCAGCCTGGCCGCGCTGACCGCGCACCAGGCCTGCCACCTG
HisHisHisLeuGluGlyGlySerLeuAlaAlaLeuThrAlaHisGlnAlaCysHisLeu
190 210 230
CCGCTGGAGACTTTCACCCGTCATCGCCAGCCGCGCGGCTGGGAACAACTGGAGCAGTGC
ProLeuGluThrPheThrArgHisArgGlnProArgGlyTrpGluGlnLeuGluGlnCys
250 270 290
GGCTATCCGGTGCAGCGGCTGGTCGCCCTCTACCTGGCGGCGCGACTGTCATGGAACCAG
GlyTyrProValGlnArgLeuValAlaLeuTyrLeuAlaAlaArgLeuSerTrpAsnGln
310 330 350
GTCGACCAGGTGATCCGCAACGCCCTGGCCAGCCCCGGCAGCGGCGGCGACCTGGGCGAA
ValAspGlnValIleArgAsnAlaLeuAlaSerProGlySerGlyGlyAspLeuGlyGlu
370 390 410
GCGATCCGCGAGCAGCCGGAGCAGGCCCGTCTGGCCCTGACCCTGGCCGCCGCCGAGAGC
AlaIleArgGluGlnProGluGlnAlaArgLeuAlaLeuThrLeuAlaAlaAlaGluSer
430 450 470
GAGCGCTTCGTCCGGCAGGGCACCGGCAACGACGAGGCCGGCGCGGCCAACGCCGACGAG
GluArgPheValArgGlnGlyThrGlyAsnAspGluAlaGlyAlaAlaAsnAlaAspGlu
490 510 530
AAGCTTCAGGTACAACTGCAGCAGTCTGGACCTGAACTGAAGAAGCCTGGAGAGACAGTC
LysLeuGlnValGlnLeuGlnGlnSerGlyProGluLeuLysLysProGlyGluThrVal
550 570 590
AAGATCTCCTGCAAGGCCTCTGGGTATCCTTTCACAAACTATGGAATGAACTGGGTGAAG
LysIleSerCysLysAlaSerGlyTyrProPheThrAsnTyrGlyMetAsnTrpValLys
610 630 650
CAGGCTCCAGGACAGGGTTTAAAGTGGATGGGCTGGATTAACACCTCCACTGGAGAGTCA
GlnAlaProGlyGlnGlyLeuLysTrpMetGlyTrpIleAsnThrSerThrGlyGluSer
670 690 710
ACATTTGCTGATGACTTCAAGGGACGGTTTGACTTCTCTTTGGAAACCTCTGCCAACACT
ThrPheAlaAspAspPheLysGlyArgPheAspPheSerLeuGluThrSerAlaAsnThr
730 750 770
GCCTATTTGCAGATCAACAACCTCAAAAGTGAAGACATGGCTACATATTTCTGTGCAAGA
AlaTyrLeuGlnIleAsnAsnLeuLysSerGluAspMetAlaThrTyrPheCysAlaArg
790 810 830
TGGGAGGTTTACCACGGCTACGTTCCTTACTGGGGCCAAGGGACCACGGTCACCGTTTCC
TrpGluValTyrHisGlyTyrValProTyrTrpGlyGlnGlyThrThrValThrValSer

850 870 890
TCTGGCGGTGGCGGTTCTGGTGGCGGTGGCTCCGGCGGTGGCGGTTCTGACATCCAGCTG
SerGlyGlyGlyGlySerGlyGlyGlyGlySerGlyGlyGlyGlySerAspIleGlnLeu
910 930 950
ACCCAGTCTCACAAATTCCTGTCCACTTCAGTAGGAGACAGGGTCAGCATCACCTGCAAG
ThrGlnSerHisLysPheLeuSerThrSerValGlyAspArgValSerIleThrCysLys
970 990 1010
GCCAGTCAGGATGTGTATAATGCTGTTGCCTGGTATCAACAGAAACCAGGACAATCTCCT
AlaSerGlnAspValTyrAsnAlaValAlaTrpTyrGlnGlnLysProGlyGlnSerPro
1030 1050 1070
AAACTTCTGATTTACTCGGCATCCTCCCGGTACACTGGAGTCCCTTCTCGCTTCACTGGC
LysLeuLeuIleTyrSerAlaSerSerArgTyrThrGlyValProSerArgPheThrGly
1090 1110 1130
AGTGGCTCTGGGCCGGATTTCACTTTCACCATCAGCAGTGTGCAGGCTGAAGACCTGGCA
SerGlySerGlyProAspPheThrPheThrIleSerSerValGlnAlaGluAspLeuAla
1150 1170 1190
GTTTATTTCTGTCAGCAACATTTTCGTACTCCATTCACGTTCGGCTCGGGGACAAAATTG
ValTyrPheCysGlnGlnHisPheArgThrProPheThrPheGlySerGlyThrLysLeu
1210 1230 1250
GAGATCAAAGCTCTAGAGGATCCCCGGGTACCGAGCTCTGCGGGCCCGGCGGACAGCGGC
GluIleLysAlaLeuGluAspProArgValProSerSerAlaGlyProAlaAspSerGly
1270 1290 1310
GACGCCCTGCTGGAGCGCAACTATCCCACTGGCGCGGAGTTCCTCGGCGACGGCGGCGAC
AspAlaLeuLeuGluArgAsnTyrProThrGlyAlaGluPheLeuGlyAspGlyGlyAsp
1330 1350 1370
GTCAGCTTCAGCACCCGCGGCACGCAGAACTGGACGGTGGAGCGGCTGCTCCAGGCGCAC
ValSerPheSerThrArgGlyThrGlnAsnTrpThrValGluArgLeuLeuGlnAlaHis
1390 1410 1430
CGCCAACTGGAGGAGCGCGGCTATGTGTTCGTCGGCTACCACGGCACCTTCCTCGAAGCG
ArgGlnLeuGluGluArgGlyTyrValPheValGlyTyrHisGlyThrPheLeuGluAla
1450 1470 1490
GCGCAAAGCATCGTCTTCGGCGGGGTGCGCGCGCGCAGCCAGGACCTCGACGCGATCTGG
AlaGlnSerIleValPheGlyGlyValArgAlaArgSerGlnAspLeuAspAlaIleTrp
1510 1530 1550
CGCGGTTTCTATATCGCCGGCGATCCGGCGCTGGCCTACGGCTACGCCCAGGACCAGGAA
ArgGlyPheTyrIleAlaGlyAspProAlaLeuAlaTyrGlyTyrAlaGlnAspGlnGlu
1570 1590 1610
CCCGACGCACGCGGCCGGATCCGCAACGGTGCCCTGCTGCGGGTCTATGTGCCGCGCTCG
ProAspAlaArgGlyArgIleArgAsnGlyAlaLeuLeuArgValTyrValProArgSer
1630 1650 1670
AGCCTGCCGGGCTTCTACCGCACCAGCCTGACCCTGGCCGCGCCGGAGGCGGCGGGCGAG
SerLeuProGlyPheTyrArgThrSerLeuThrLeuAlaAlaProGluAlaAlaGlyGlu
1690 1710 1730
GTCGAACGGCTGATCGGCCATCCGCTGCCGCTGCGCCTGGACGCCATCACCGGCCCCGAG
ValGluArgLeuIleGlyHisProLeuProLeuArgLeuAspAlaIleThrGlyProGlu
1750 1770 1790
GAGGAAGGCGGGCGCCTGGAGACCATTCTCGGCTGGCCGCTGGCCGAGCGCACCGTGGTG
GluGluGlyGlyArgLeuGluThrIleLeuGlyTrpProLeuAlaGluArgThrValVal
1810 1830 1850
ATTCCCTCGGCGATCCCCACCGACCCGCGCAACGTCGGCGGCGACCTCGACCCGTCCAGC
IleProSerAlaIleProThrAspProArgAsnValGlyGlyAspLeuAspProSerSer
1870 1890 1910
ATCCCCGACAAGGAACAGGCGATCAGCGCCCTGCCGGACTACGCCAGCCAGCCCGGCAAA
IleProAspLysGluGlnAlaIleSerAlaLeuProAspTyrAlaSerGlnProGlyLys
1930 1950 1970
CCGCCGCGCGAAGCTTCTCAGGTACAACTGCAGCAGTCTGGACCTGAACTGAAGAAGCCT
ProProArgGluAlaSerGlnValGlnLeuGlnGlnSerGlyProGluLeuLysLysPro
1990 2010 2030
GGAGAGACAGTCAAGATCTCCTGCAAGGCCTCTGGGTATCCTTTCACAAACTATGGAATG
GlyGluThrValLysIleSerCysLysAlaSerGlyTyrProPheThrAsnTyrGlyMet
2050 2070 2090
AACTGGGTGAAGCAGGCTCCAGGACAGGGTTTAAAGTGGATGGGCTGGATTAACACCTCC
AsnTrpValLysGlnAlaProGlyGlnGlyLeuLysTrpMetGlyTrpIleAsnThrSer

2110 2130 2150
ACTGGAGAGTCAACATTTGCTGATGACTTCAAGGGACGGTTTGACTTCTCTTTGGAAACC
ThrGlyGluSerThrPheAlaAspAspPheLysGlyArgPheAspPheSerLeuGluThr
2170 2190 2210
TCTGCCAACACTGCCTATTTGCAGATCAACAACCTCAAAAGTGAAGACATGGCTACATAT
SerAlaAsnThrAlaTyrLeuGlnIleAsnAsnLeuLysSerGluAspMetAlaThrTyr
2230 2250 2270
TTCTGTGCAAGATGGGAGGTTTACCACGGCTACGTTCCTTACTGGGGCCAAGGGACCACG
PheCysAlaArgTrpGluValTyrHisGlyTyrValProTyrTrpGlyGlnGlyThrThr
2290 2310 2330
GTCACCGTTTCCTCTGGCGGTGGCGGTTCTGGTGGCGGTGGCTCCGGCGGTGGCGGTTCT
ValThrValSerSerGlyGlyGlyGlySerGlyGlyGlyGlySerGlyGlyGlyGlySer
2350 2370 2390
GACATCCAGCTGACCCAGTCTCACAAATTCCTGTCCACTTCAGTAGGAGACAGGGTCAGC
AspIleGlnLeuThrGlnSerHisLysPheLeuSerThrSerValGlyAspArgValSer
2410 2430 2450
ATCACCTGCAAGGCCAGTCAGGATGTGTATAATGCTGTTGCCTGGTATCAACAGAAACCA
IleThrCysLysAlaSerGlnAspValTyrAsnAlaValAlaTrpTyrGlnGlnLysPro
2470 2490 2510
GGACAATCTCCTAAACTTCTGATTTACTCGGCATCCTCCCGGTACACTGGAGTCCCTTCT
GlyGlnSerProLysLeuLeuIleTyrSerAlaSerSerArgTyrThrGlyValProSer
2530 2550 2570
CGCTTCACTGGCAGTGGCTCTGGGCCGGATTTCACTTTCACCATCAGCAGTGTGCAGGCT
ArgPheThrGlySerGlySerGlyProAspPheThrPheThrIleSerSerValGlnAla
2590 2610 2630
GAAGACCTGGCAGTTTATTTCTGTCAGCAACATTTTCGTACTCCATTCACGTTCGGCTCG
GluAspLeuAlaValTyrPheCysGlnGlnHisPheArgThrProPheThrPheGlySer
2650 2670 2690
GGGACAAAATTGGAGATCAAAGCTCTAGGCGAGAAAGATGAACTTTAATCTAGAGGTACC
GlyThrLysLeuGluIleLysAlaLeuGlyGluLysAspGluLeuEnd
2710
GAGCTCGAATTCGTCGAG

SEQ ID NO: 21 and 22
SEQUENCE TYPE: nucleotide
SEQUENCE LENGTH: 2793 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pMS238-5-5

FEATURES: from 1 to 63 bp ompA signal peptide
from 64 to 114 bp synthetic spacer
from 115 to 834 bp scFv(FRP5) cDNA
from 835 to 867 bp synthetic spacer
from 868 to 1212 bp ETA gene amino acids 252 to 366
from 1213 to 1221 bp synthetic spacer
from 1222 to 1941 bp scFv(FRP5) cDNA
from 1942 to 1974 bp synthetic spacer
from 1975 to 2679 bp ETA gene amino acids 380 to 613

PROPERTIES: encodes scFv2(FRP5/FRP5)-ETA (version 2)

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGCAGTCTGGACCTGAACTGAAGAAGCCTGGAGAGACAGTCAAGATCTCCTGC
GlnLeuGlnGlnSerGlyProGluLeuLysLysProGlyGluThrValLysIleSerCys
190 210 230
AAGGCCTCTGGGTATCCTTTCACAAACTATGGAATGAACTGGGTGAAGCAGGCTCCAGGA
LysAlaSerGlyTyrProPheThrAsnTyrGlyMetAsnTrpValLysGlnAlaProGly
250 270 290
CAGGGTTTAAAGTGGATGGGCTGGATTAACACCTCCACTGGAGAGTCAACATTTGCTGAT
GlnGlyLeuLysTrpMetGlyTrpIleAsnThrSerThrGlyGluSerThrPheAlaAsp
310 330 350
GACTTCAAGGGACGGTTTGACTTCTCTTTGGAAACCTCTGCCAACACTGCCTATTTGCAG
AspPheLysGlyArgPheAspPheSerLeuGluThrSerAlaAsnThrAlaTyrLeuGln
370 390 410
ATCAACAACCTCAAAAGTGAAGACATGGCTACATATTTCTGTGCAAGATGGGAGGTTTAC
IleAsnAsnLeuLysSerGluAspMetAlaThrTyrPheCysAlaArgTrpGluValTyr
430 450 470
CACGGCTACGTTCCTTACTGGGGCCAAGGGACCACGGTCACCGTTTCCTCTGGCGGTGGC
HisGlyTyrValProTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCAC
GlySerGlyGlyGlyGlySerGlyGlyGlyGlySerAspIleGlnLeuThrGlnSerHis
550 570 590
AAATTCCTGTCCACTTCAGTAGGAGACAGGGTCAGCATCACCTGCAAGGCCAGTCAGGAT
LysPheLeuSerThrSerValGlyAspArgValSerIleThrCysLysAlaSerGlnAsp
610 630 650
GTGTATAATGCTGTTGCCTGGTATCAACAGAAACCAGGACAATCTCCTAAACTTCTGATT
ValTyrAsnAlaValAlaTrpTyrGlnGlnLysProGlyGlnSerProLysLeuLeuIle
670 690 710
TACTCGGCATCCTCCCGGTACACTGGAGTCCCTTCTCGCTTCACTGGCAGTGGCTCTGGG
TyrSerAlaSerSerArgTyrThrGlyValProSerArgPheThrGlySerGlySerGly
730 750 770
CCGGATTTCACTTTCACCATCAGCAGTGTGCAGGCTGAAGACCTGGCAGTTTATTTCTGT
ProAspPheThrPheThrIleSerSerValGlnAlaGluAspLeuAlaValTyrPheCys
790 810 830
CAGCAACATTTTCGTACTCCATTCACGTTCGGCTCGGGGACAAAATTGGAGATCAAAGCT
GlnGlnHisPheArgThrProPheThrPheGlySerGlyThrLysLeuGluIleLysAla

850 870 890
CTAGAGCACCATCATCACCATCACCTAGAGGGCGGCAGCCTGGCCGCGCTGACCGCGCAC
LeuGluHisHisHisHisHisHisLeuGluGlyGlySerLeuAlaAlaLeuThrAlaHis
910 930 950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970 990 1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030 1050 1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090 1110 1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150 1170 1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210 1230 1250
GCCAACGCCGACGAGAAGCTTCAGGTACAACTGCAGCAGTCTGGACCTGAACTGAAGAAG
AlaAsnAlaAspGluLysLeuGlnValGlnLeuGlnGlnSerGlyProGluLeuLysLys
1270 1290 1310
CCTGGAGAGACAGTCAAGATCTCCTGCAAGGCCTCTGGGTATCCTTTCACAAACTATGGA
ProGlyGluThrValLysIleSerCysLysAlaSerGlyTyrProPheThrAsnTyrGly
1330 1350 1370
ATGAACTGGGTGAAGCAGGCTCCAGGACAGGGTTTAAAGTGGATGGGCTGGATTAACACC
MetAsnTrpValLysGlnAlaProGlyGlnGlyLeuLysTrpMetGlyTrpIleAsnThr
1390 1410 1430
TCCACTGGAGAGTCAACATTTGCTGATGACTTCAAGGGACGGTTTGACTTCTCTTTGGAA
SerThrGlyGluSerThrPheAlaAspAspPheLysGlyArgPheAspPheSerLeuGlu
1450 1470 1490
ACCTCTGCCAACACTGCCTATTTGCAGATCAACAACCTCAAAAGTGAAGACATGGCTACA
ThrSerAlaAsnThrAlaTyrLeuGlnIleAsnAsnLeuLysSerGluAspMetAlaThr
1510 1530 1550
TATTTCTGTGCAAGATGGGAGGTTTACCACGGCTACGTTCCTTACTGGGGCCAAGGGACC
TyrPheCysAlaArgTrpGluValTyrHisGlyTyrValProTyrTrpGlyGlnGlyThr
1570 1590 1610
ACGGTCACCGTTTCCTCTGGCGGTGGCGGTTCTGGTGGCGGTGGCTCCGGCGGTGGCGGT
ThrValThrValSerSerGlyGlyGlyGlySerGlyGlyGlyGlySerGlyGlyGlyGly
1630 1650 1670
TCTGACATCCAGCTGACCCAGTCTCACAAATTCCTGTCCACTTCAGTAGGAGACAGGGTC
SerAspIleGlnLeuThrGlnSerHisLysPheLeuSerThrSerValGlyAspArgVal
1690 1710 1730
AGCATCACCTGCAAGGCCAGTCAGGATGTGTATAATGCTGTTGCCTGGTATCAACAGAAA
SerIleThrCysLysAlaSerGlnAspValTyrAsnAlaValAlaTrpTyrGlnGlnLys
1750 1770 1790
CCAGGACAATCTCCTAAACTTCTGATTTACTCGGCATCCTCCCGGTACACTGGAGTCCCT
ProGlyGlnSerProLysLeuLeuIleTyrSerAlaSerSerArgTyrThrGlyValPro
1810 1830 1850
TCTCGCTTCACTGGCAGTGGCTCTGGGCCGGATTTCACTTTCACCATCAGCAGTGTGCAG
SerArgPheThrGlySerGlySerGlyProAspPheThrPheThrIleSerSerValGln
1870 1890 1910
GCTGAAGACCTGGCAGTTTATTTCTGTCAGCAACATTTTCGTACTCCATTCACGTTCGGC
AlaGluAspLeuAlaValTyrPheCysGlnGlnHisPheArgThrProPheThrPheGly
1930 1950 1970
TCGGGGACAAAATTGGAGATCAAAGCTCTAGAGGATCCCCGGGTACCGAGCTCTGCGGGC
SerGlyThrLysLeuGluIleLysAlaLeuGluAspProArgValProSerSerAlaGly
1990 2010 2030
CCGGCGGACAGCGGCGACGCCCTGCTGGAGCGCAACTATCCCACTGGCGCGGAGTTCCTC
ProAlaAspSerGlyAspAlaLeuLeuGluArgAsnTyrProThrGlyAlaGluPheLeu
2050 2070 2090
GGCGACGGCGGCGACGTCAGCTTCAGCACCCGCGGCACGCAGAACTGGACGGTGGAGCGG
GlyAspGlyGlyAspValSerPheSerThrArgGlyThrGlnAsnTrpThrValGluArg

2110                    2130                    2150
CTGCTCCAGGCGCACCGCCAACTGGAGGAGCGCGGCTATGTGTTCGTCGGCTACCACGGC
LeuLeuGlnAlaHisArgGlnLeuGluGluArgGlyTyrValPheValGlyTyrHisGly
2170                    2190                    2210
ACCTTCCTCGAAGCGGCGCAAAGCATCGTCTTCGGCGGGGTGCGCGCGCAGCCAGGAC
ThrPheLeuGluAlaAlaGlnSerIleValPheGlyGlyValArgAlaArgSerGlnAsp
2230                    2250                    2270
CTCGACGCGATCTGGCGCGGTTTCTATATCGCCGGCGATCCGGCGCTGGCCTACGGCTAC
LeuAspAlaIleTrpArgGlyPheTyrIleAlaGlyAspProAlaLeuAlaTyrGlyTyr
2290                    2310                    2330
GCCCAGGACCAGGAACCCGACGCACGCGGCCGGATCCGCAACGGTGCCCTGCTGCGGGTC
AlaGlnAspGlnGluProAspAlaArgGlyArgIleArgAsnGlyAlaLeuLeuArgVal
2350                    2370                    2390
TATGTGCCGCGCTCGAGCCTGCCGGGCTTCTACCGCACCAGCCTGACCCTGGCCGCGCCG
TyrValProArgSerSerLeuProGlyPheTyrArgThrSerLeuThrLeuAlaAlaPro
2410                    2430                    2450
GAGGCGGCGGGCGAGGTCGAACGGCTGATCGGCCATCCGCTGCCGCTGCGCCTGGACGCC
GluAlaAlaGlyGluValGluArgLeuIleGlyHisProLeuProLeuArgLeuAspAla
2470                    2490                    2510
ATCACCGGCCCCGAGGAGGAAGGCGGGCGCCTGGAGACCATTCTCGGCTGGCCGCTGGCC
IleThrGlyProGluGluGluGlyGlyArgLeuGluThrIleLeuGlyTrpProLeuAla
2530                    2550                    2570
GAGCGCACCGTGGTGATTCCCTCGGCGATCCCCACCGACCCGCGCAACGTCGGCGGCGAC
GluArgThrValValIleProSerAlaIleProThrAspProArgAsnValGlyGlyAsp
2590                    2610                    2630
CTCGACCCGTCCAGCATCCCCGACAAGGAACAGGCGATCAGCGCCCTGCCGGACTACGCC
LeuAspProSerSerIleProAspLysGluGlnAlaIleSerAlaLeuProAspTyrAla
2650                    2670                    2690
AGCCAGCCCGGCAAACCGCCGCGCGAGGACCTGAAGTAACTGCCGCGACCGGCCGGCTCC
SerGlnProGlyLysProProArgGluAspLeuLysEnd
2710                    2730                    2750
CTTCGCAGGAGCCGGCCTTCTCGGGGCCTGGCCATACATCAGGTTTTCCTGATGCCAGCC
2770                    2790
CAATCGAATATGAATTTCGACAGATCTCTCGAG

SEQ ID NO: 23 and 24
SEQUENCE TYPE: nucleotide
SEQUENCE LENGTH: 2730 bp
MOLECULE TYPE: plasmid DNA
ORIGINAL SOURCE ORGANISM: mouse; P. aeruginosa; synthetic
IMMEDIATE EXPERIMENTAL SOURCE: E.coli
NAME OF CELL CLONE: pMS246-5-5 KDEL

FEATURES: from 1 to 63 bp ompA signal peptide
from 64 to 114 bp synthetic spacer
from 115 to 834 bp scFv(FRP5) cDNA
from 835 to 867 bp synthetic spacer
from 868 to 1944 bp ETA gene amino acids 252 to 610
from 1945 to 1950 bp synthetic spacer
from 1951 to 2670 bp scFv(FRP5) cDNA
from 2671 to 2700 bp synthetic spacer

PROPERTIES: encodes scFv2(FRP5/FRP5)-ETA (version 3)

10 30 50
ATGAAAAAGACAGCTATCGCGATTGCAGTGGCACTGGCTGGTTTCGCTACCGTTGCGCAA
MetLysLysThrAlaIleAlaIleAlaValAlaLeuAlaGlyPheAlaThrValAlaGln
70 90 110
GCTGACTACAAGGACGACGATGACAAGCTGCACCATCATCACCATCACAAGCTTCAGGTA
AlaAspTyrLysAspAspAspAspLysLeuHisHisHisHisHisHisLysLeuGlnVal
130 150 170
CAACTGCAGCAGTCTGGACCTGAACTGAAGAAGCCTGGAGAGACAGTCAAGATCTCCTGC
GlnLeuGlnGlnSerGlyProGluLeuLysLysProGlyGluThrValLysIleSerCys
190 210 230
AAGGCCTCTGGGTATCCTTTCACAAACTATGGAATGAACTGGGTGAAGCAGGCTCCAGGA
LysAlaSerGlyTyrProPheThrAsnTyrGlyMetAsnTrpValLysGlnAlaProGly
250 270 290
CAGGGTTTAAAGTGGATGGGCTGGATTAACACCTCCACTGGAGAGTCAACATTTGCTGAT
GlnGlyLeuLysTrpMetGlyTrpIleAsnThrSerThrGlyGluSerThrPheAlaAsp
310 330 350
GACTTCAAGGGACGGTTTGACTTCTCTTTGGAAACCTCTGCCAACACTGCCTATTTGCAG
AspPheLysGlyArgPheAspPheSerLeuGluThrSerAlaAsnThrAlaTyrLeuGln
370 390 410
ATCAACAACCTCAAAAGTGAAGACATGGCTACATATTTCTGTGCAAGATGGGAGGTTTAC
IleAsnAsnLeuLysSerGluAspMetAlaThrTyrPheCysAlaArgTrpGluValTyr
430 450 470
CACGGCTACGTTCCTTACTGGGGCCAAGGGACCACGGTCACCGTTTCCTCTGGCGGTGGC
HisGlyTyrValProTyrTrpGlyGlnGlyThrThrValThrValSerSerGlyGlyGly
490 510 530
GGTTCTGGTGGCGGTGGCTCCGGCGGTGGCGGTTCTGACATCCAGCTGACCCAGTCTCAC
GlySerGlyGlyGlyGlySerGlyGlyGlyGlySerAspIleGlnLeuThrGlnSerHis
550 570 590
AAATTCCTGTCCACTTCAGTAGGAGACAGGGTCAGCATCACCTGCAAGGCCAGTCAGGAT
LysPheLeuSerThrSerValGlyAspArgValSerIleThrCysLysAlaSerGlnAsp
610 630 650
GTGTATAATGCTGTTGCCTGGTATCAACAGAAACCAGGACAATCTCCTAAACTTCTGATT
ValTyrAsnAlaValAlaTrpTyrGlnGlnLysProGlyGlnSerProLysLeuLeuIle
670 690 710
TACTCGGCATCCTCCCGGTACACTGGAGTCCCTTCTCGCTTCACTGGCAGTGGCTCTGGG
TyrSerAlaSerSerArgTyrThrGlyValProSerArgPheThrGlySerGlySerGly
730 750 770
CCGGATTTCACTTTCACCATCAGCAGTGTGCAGGCTGAAGACCTGGCAGTTTATTTCTGT
ProAspPheThrPheThrIleSerSerValGlnAlaGluAspLeuAlaValTyrPheCys
790 810 830
CAGCAACATTTTCGTACTCCATTCACGTTCGGCTCGGGGACAAAATTGGAGATCAAAGCT
GlnGlnHisPheArgThrProPheThrPheGlySerGlyThrLysLeuGluIleLysAla

850 870 890
CTAGAGCACCATCATCACCATCACCTAGAGGGCGGCAGCCTGGCCGCGCTGACCGCGCAC
LeuGluHisHisHisHisHisHisLeuGluGlyGlySerLeuAlaAlaLeuThrAlaHis
910 930 950
CAGGCCTGCCACCTGCCGCTGGAGACTTTCACCCGTCATCGCCAGCCGCGCGGCTGGGAA
GlnAlaCysHisLeuProLeuGluThrPheThrArgHisArgGlnProArgGlyTrpGlu
970 990 1010
CAACTGGAGCAGTGCGGCTATCCGGTGCAGCGGCTGGTCGCCCTCTACCTGGCGGCGCGA
GlnLeuGluGlnCysGlyTyrProValGlnArgLeuValAlaLeuTyrLeuAlaAlaArg
1030 1050 1070
CTGTCATGGAACCAGGTCGACCAGGTGATCCGCAACGCCCTGGCCAGCCCCGGCAGCGGC
LeuSerTrpAsnGlnValAspGlnValIleArgAsnAlaLeuAlaSerProGlySerGly
1090 1110 1130
GGCGACCTGGGCGAAGCGATCCGCGAGCAGCCGGAGCAGGCCCGTCTGGCCCTGACCCTG
GlyAspLeuGlyGluAlaIleArgGluGlnProGluGlnAlaArgLeuAlaLeuThrLeu
1150 1170 1190
GCCGCCGCCGAGAGCGAGCGCTTCGTCCGGCAGGGCACCGGCAACGACGAGGCCGGCGCG
AlaAlaAlaGluSerGluArgPheValArgGlnGlyThrGlyAsnAspGluAlaGlyAla
1210 1230 1250
GCCAACGCCGACGTGGTGAGCCTGACCTGCCCGGTCGCCGCCGGTGAATGCGCGGGCCCG
AlaAsnAlaAspValValSerLeuThrCysProValAlaAlaGlyGluCysAlaGlyPro
1270 1290 1310
GCGGACAGCGGCGACGCCCTGCTGGAGCGCAACTATCCCACTGGCGCGGAGTTCCTCGGC
AlaAspSerGlyAspAlaLeuLeuGluArgAsnTyrProThrGlyAlaGluPheLeuGly
1330 1350 1370
GACGGCGGCGACGTCAGCTTCAGCACCCGCGGCACGCAGAACTGGACGGTGGAGCGGCTG
AspGlyGlyAspValSerPheSerThrArgGlyThrGlnAsnTrpThrValGluArgLeu
1390 1410 1430
CTCCAGGCGCACCGCCAACTGGAGGAGCGCGGCTATGTGTTCGTCGGCTACCACGGCACC
LeuGlnAlaHisArgGlnLeuGluGluArgGlyTyrValPheValGlyTyrHisGlyThr
1450 1470 1490
TTCCTCGAAGCGGCGCAAAGCATCGTCTTCGGCGGGGTGCGCGCGCGCAGCCAGGACCTC
PheLeuGluAlaAlaGlnSerIleValPheGlyGlyValArgAlaArgSerGlnAspLeu
1510 1530 1550
GACGCGATCTGGCGCGGTTTCTATATCGCCGGCGATCCGGCGCTGGCCTACGGCTACGCC
AspAlaIleTrpArgGlyPheTyrIleAlaGlyAspProAlaLeuAlaTyrGlyTyrAla
1570 1590 1610
CAGGACCAGGAACCCGACGCACGCGGCCGGATCCGCAACGGTGCCCTGCTGCGGGTCTAT
GlnAspGlnGluProAspAlaArgGlyArgIleArgAsnGlyAlaLeuLeuArgValTyr
1630 1650 1670
GTGCCGCGCTCGAGCCTGCCGGGCTTCTACCGCACCAGCCTGACCCTGGCCGCGCCGGAG
ValProArgSerSerLeuProGlyPheTyrArgThrSerLeuThrLeuAlaAlaProGlu
1690 1710 1730
GCGGCGGGCGAGGTCGAACGGCTGATCGGCCATCCGCTGCCGCTGCGCCTGGACGCCATC
AlaAlaGlyGluValGluArgLeuIleGlyHisProLeuProLeuArgLeuAspAlaIle
1750 1770 1790
ACCGGCCCCGAGGAGGAAGGCGGGCGCCTGGAGACCATTCTCGGCTGGCCGCTGGCCGAG
ThrGlyProGluGluGluGlyGlyArgLeuGluThrIleLeuGlyTrpProLeuAlaGlu
1810 1830 1850
CGCACCGTGGTGATTCCCTCGGCGATCCCCACCGACCCGCGCAACGTCGGCGGCGACCTC
ArgThrValValIleProSerAlaIleProThrAspProArgAsnValGlyGlyAspLeu
1870 1890 1910
GACCCGTCCAGCATCCCCGACAAGGAACAGGCGATCAGCGCCCTGCCGGACTACGCCAGC
AspProSerSerIleProAspLysGluGlnAlaIleSerAlaLeuProAspTyrAlaSer
1930 1950 1970
CAGCCCGGCAAACCGCCGCGCGAAGCTTCTCAGGTACAACTGCAGCAGTCTGGACCTGAA
GlnProGlyLysProProArgGluAlaSerGlnValGlnLeuGlnGlnSerGlyProGlu
1990 2010 2030
CTGAAGAAGCCTGGAGAGACAGTCAAGATCTCCTGCAAGGCCTCTGGGTATCCTTTCACA
LeuLysLysProGlyGluThrValLysIleSerCysLysAlaSerGlyTyrProPheThr
2050 2070 2090
AACTATGGAATGAACTGGGTGAAGCAGGCTCCAGGACAGGGTTTAAAGTGGATGGGCTGG
AsnTyrGlyMetAsnTrpValLysGlnAlaProGlyGlnGlyLeuLysTrpMetGlyTrp

2110 2130 2150
ATTAACACCTCCACTGGAGAGTCAACATTTGCTGATGACTTCAAGGGACGGTTTGACTTC
IleAsnThrSerThrGlyGluSerThrPheAlaAspAspPheLysGlyArgPheAspPhe
2170 2190 2210
TCTTTGGAAACCTCTGCCAACACTGCCTATTTGCAGATCAACAACCTCAAAAGTGAAGAC
SerLeuGluThrSerAlaAsnThrAlaTyrLeuGlnIleAsnAsnLeuLysSerGluAsp
2230 2250 2270
ATGGCTACATATTTCTGTGCAAGATGGGAGGTTTACCACGGCTACGTTCCTTACTGGGGC
MetAlaThrTyrPheCysAlaArgTrpGluValTyrHisGlyTyrValProTyrTrpGly
2290 2310 2330
CAAGGGACCACGGTCACCGTTTCCTCTGGCGGTGGCGGTTCTGGTGGCGGTGGCTCCGGC
GlnGlyThrThrValThrValSerSerGlyGlyGlyGlySerGlyGlyGlyGlySerGly
2350 2370 2390
GGTGGCGGTTCTGACATCCAGCTGACCCAGTCTCACAAATTCCTGTCCACTTCAGTAGGA
GlyGlyGlySerAspIleGlnLeuThrGlnSerHisLysPheLeuSerThrSerValGly
2410 2430 2450
GACAGGGTCAGCATCACCTGCAAGGCCAGTCAGGATGTGTATAATGCTGTTGCCTGGTAT
AspArgValSerIleThrCysLysAlaSerGlnAspValTyrAsnAlaValAlaTrpTyr
2470 2490 2510
CAACAGAAACCAGGACAATCTCCTAAACTTCTGATTTACTCGGCATCCTCCCGGTACACT
GlnGlnLysProGlyGlnSerProLysLeuLeuIleTyrSerAlaSerSerArgTyrThr
2530 2550 2570
GGAGTCCCTTCTCGCTTCACTGGCAGTGGCTCTGGGCCGGATTTCACTTTCACCATCAGC
GlyValProSerArgPheThrGlySerGlySerGlyProAspPheThrPheThrIleSer
2590 2610 2630
AGTGTGCAGGCTGAAGACCTGGCAGTTTATTTCTGTCAGCAACATTTTCGTACTCCATTC
SerValGlnAlaGluAspLeuAlaValTyrPheCysGlnGlnHisPheArgThrProPhe
2650 2670 2690
ACGTTCGGCTCGGGGACAAAATTGGAGATCAAAGCTCTAGGCGAGAAAGATGAACTTTAA
ThrPheGlySerGlyThrLysLeuGluIleLysAlaLeuGlyGluLysAspGluLeuEnd
2710 2730
TCTAGAGGTACCGAGCTCGAATTCGTCGAG

## Claims

1. A recombinant fusion polypeptide comprising at least two independent cell surface binding domains, at least one of which binds to a member of the EGF receptor family of growth factor receptors.

2. The polypeptide of Claim 1 comprising two binding domains which bind to a member of the EGF receptor family of growth factor receptors.

3. The polypeptide of Claim 1 or 2 wherein the member of the EGF receptor family of growth factor receptors is the EGF receptor, erbB-2, erbB-3 or erb-B4.

4. The polypeptide of any one of Claims 1-3 wherein at least one binding domain comprises a single chain binding region of an antibody.

5. The polypeptide of Claim 4 wherein the binding region of an antibody comprises the variable heavy chain sequence and the variable light chain sequence of the antibody connected by a peptide linker.

6. The polypeptide of Claim 5 wherein the linker comprises 5 to 25 amino acids.

7. The polypeptide of any one of Claims 1-6 wherein at least one binding domain comprises the binding region of a growth factor.

8. The polypeptide of any one of Claims 1-7 wherein at least one domain comprises the binding region of an antibody and at least one domain comprises the binding region of a growth factor.

9. The polypeptide of Claim 4 wherein at least two domains comprise the binding regions of an antibody.

10. The polypeptide of Claim 7 wherein at least two domains comprise the binding regions of a growth factor.

11. The polypeptide of Claim 4 that comprises the binding region of monoclonal antibody 225.

12. The polypeptide of Claim 4 that comprises the binding region of monoclonal antibody FRP5, monoclonal antibody FSP16, monoclonal antibody FSP77, or monoclonal antibody FWP51.

13. The polypeptide of Claim 7 that comprises the binding region of growth factor EGF, growth factor TGF-alpha, heregulin, Neu differentiation factor, amphiregulin, heparin-binding EGF, or cripto-l.

14. The polypeptide of Claim 13 that comprises the binding region of growth factor EGF or growth factor TGF-alpha.

15. The polypeptide of Claim 8 that comprises the binding region of monoclonal antibody FRP5, monoclonal antibody FSP16, monoclonal antibody FSP77, or monoclonal antibody FWP51 and the binding region of growth factor EGF or growth factor TGF-alpha.

16. The polypeptide of Claim 8 that comprises the binding region of monoclonal antibody 225 and the binding region of growth factor EGF or growth factor TGF-alpha.

17. The polypeptide of Clain 15 that comprises the binding region of monoclonal antibody FRP5 and the binding region of growth factor TGF- alpha.

18. The polypeptide of Claim 16 that comprises the binding region of monoclonal antibody 225 and the binding region of growth factor TGF-alpha.

19. The polypeptide of Claim 9 that comprises two binding regions of monoclonal antibody FRP5.

20. The polypeptide of Claim 9 that comprises two binding regions of monoclonal antibody 225.

21. The polypeptide of any one of Claims 1-20 further comprising at least one effector region.

22. The polypeptide of Claim 21 wherein the effector region comprises a cytotoxin.

23. The polypeptide of Claim 22 wherein the cytotoxin is derived from bacteria.

24. The polypeptide of Claim 23 wherein the cytotoxin is diphtheria toxin derived from Corynebacterium diphtheriae, exotoxin A derived from Pseudomonas aeruginosa, anthrax toxins derived from Bacillus anthracis, Shigatoxin derived from Shigella, Shiga-like toxins derived from Escherichia coli, or pertussis toxins derived from Bordetella pertussis or a toxic region thereof.

25. The polypeptide of Claim 24 wherein the cytotoxin is exotoxin A from Pseudomonas aeruginosa, or a toxic region thereof.

26. The polypeptide of Claim 25 wherein the cytotoxin comprises domains II, Ib, and III of exotoxin A from Pseudomonas aeruginosa.

27. The polypeptide of Claim 21 wherein the cytotoxin is derived from plants .

28. The polypeptide of Claim 27 wherein the cytotoxin is ricin, abrin, a ribosome inactivating protein, pokeweed antiviral protein, gelonin, or trichosanthin.

29. The polypeptide of Claim 28 wherein the ribosome inactivating protein is saporin.

30. The polypeptide of Claim 21 wherein the effector region comprises a ribonuclease derived from a mammal.

31. The polypeptide of Claim 30 wherein the mammal is a human.

32. A recombinant fusion polypeptide comprising at least two independent cell surface binding domains and at least one effector region.

33. The polypeptide of Claim 32 wherein the effector region comprises a cytotoxin.

34. A DNA molecule encoding a fusion polypeptide of any one of Claims 1-33.

35. A vector comprising at least one copy of the DNA molecule of Claim 34.

36. A host cell capable of expressing the DNA molecule of Claim 34.

37. The host cell of Claim 36 which is a bacterial cell.

38. The host cell of Claim 37 which is an E.coli cell.

39. A pharmaceutical composition comprising as an active substance a pharmaceutically effective amount of a polypeptide of any one of Claims 1-33 optionally in combination with pharmaceutically acceptable carriers, diluents and adjuvants.

40. A process for producing an anti-tumor agent comprising as an active substance a pharmaceutically effective amount of a polypeptide of any one of Claims 1-33 optionally in combination with pharmaceutically acceptable carriers, diluents and adjuvants.

41. A method for inhibiting the growth of a tumor comprising treating the tumor with a pharmaceutically effective amount of a polypeptide of any one of Claims 1-33.
